# EUROPEAN PATENT APPLICATION

(11) **EP 4 782 532 A1**
(43) Date of publication of application: **29.07.2026**
(21) Application number: 24867957.3
(22) Date of filing: 07.08.2024
(51) Int. Cl.: C12N 1/21, C12N 15/53, C12P 7/16

(54) **BUTANOL-GENERATING BACTERIUM TRANSFORMANT OF GENUS HYDROGENOPHILUS**

(30) Priority: 22.09.2023 JP 2023159281
(71) Applicant: Utilization of Carbon Dioxide Institute Co.,Ltd., Tokyo 135-0091 (JP)
(72) Inventor: YUKAWA Hideaki, Tokyo 135-0091 (JP)
(74) Representative: Kraus & Lederer PartGmbB
(86) International application number: PCT/JP2024/028273
(87) International publication number: WO 2025/062876

(57) **Abstract**

A transformant obtained by introducing, into a *Hydrogenophilus* bacterium, any one of the following genes (1) to (5) is capable of efficiently producing butanol utilizing only carbon dioxide as a carbon source.
(1) A gene encoding an enzyme having butyraldehyde dehydrogenase activity and a gene encoding an enzyme having butanol dehydrogenase activity
(2) A gene encoding a multifunctional enzyme having butyraldehyde dehydrogenase activity and butanol dehydrogenase activity
(3) A gene encoding an enzyme having butyraldehyde dehydrogenase activity and a gene encoding a multifunctional enzyme having butyraldehyde dehydrogenase activity and butanol dehydrogenase activity
(4) A gene encoding an enzyme having butanol dehydrogenase activity and a gene encoding a multifunctional enzyme having butyraldehyde dehydrogenase activity and butanol dehydrogenase activity
(5) A gene encoding an enzyme having butyraldehyde dehydrogenase activity, a gene encoding an enzyme having butanol dehydrogenase activity, and a gene encoding a multifunctional enzyme having butyraldehyde dehydrogenase activity and butanol dehydrogenase activity

## Description

### Technical Field

The present invention relates to a *Hydrogenophilus* bacterium transformant having ability to produce butanol, and to a method for producing butanol using the same.

### Background Art

The Paris Agreement, which was adopted in 2015, provides that global emissions of greenhouse gases should be promptly reduced. Under the Agreement, Japan set the goal of reducing her emission of greenhouse gases such as carbon dioxide and methane by 46% compared with year 2013 levels, by the year 2030.

Worldwide, the majority of the production of chemicals depends on petroleum resources, exacerbating the problem of increased greenhouse gas emissions. Accordingly, departure from petroleum dependency is a desirable strategy for the production of chemicals, and research and development of biorefineries that produce green chemicals from biomass is being earnestly carried out in various countries. However, the saccharification of biomass for use as raw materials of microbial fermentation necessitates complex processes, besides being costly. Using biomass that could serve as food or feed for chemical production disturbs the stable supply of food and feed. Furthermore, the large-scale consumption of biomass for chemical manufacturing raises concerns that it actually leads to environmental destruction.

As part of research geared towards departure from petroleum dependency, gases such as carbon dioxide, methane, and carbon monoxide have attracted attention as more sustainable carbon sources, and techniques for producing valuable chemicals and biofuels using microorganisms that utilize these gases are the subject of interest. In particular, carbon fixation and efficient utilization of carbon dioxide, a significant contributor to global warming, is highly anticipated.

Butanol is an organic chemical raw material with a wide range of applications in industrial sectors such as the chemical industry, the pharmaceutical industry, and the petroleum industry. Butanol is used for the production of acrylic acid esters and methacrylic acid esters used for the production of coatings, plastics, textiles, adhesives, etc.; for the production of glycol ethers used for the production of coatings and electronic engineering products; for the production of butyl acetate used for the production of paints, inks, coatings, and synthetic fruit-scented air fresheners; for the production of butylamine used for the production of pesticides and pharmaceuticals; and for the production of amine resins, etc. In addition, butanol itself has direct applications such as its use as a solvent contained in inks, dyes, etc., an extraction solvent, an antifreeze fluid, a cosmetic ingredient, or an eluent component in chromatography.

In particular, four-carbon butanol has many advantages over two-carbon ethanol, including higher fuel efficiency, easier blending with gasoline and diesel oil, low corrosion, and low water content. Consequently, butanol is attracting attention as a next-generation biofuel following bioethanol.

Butanol production methods using microorganisms include methods using acetone-butanol-ethanol fermentation (ABE fermentation) by *Clostridium* bacteria typified by *Clostridium acetobutylicum* and the like. ABE fermentation was developed by Weizmann in 1916 and can produce butanol from sugars or starches as starting materials. ABE fermentation declined primarily due to the development of the petrochemical industry in the 1950s and 1960s, but recently, growing concerns about greenhouse gas (GHG) emissions into the environment have driven a shift toward alternative fuels and renewable energy, thereby bringing renewed attention to research and development on the ABE fermentation-based production of butanol as a biofuel.

However, this method produces byproducts including solvents such as acetone and ethanol, and organic acids such as butyric acid, acetic acid, and lactic acid. These byproducts not only reduce butanol yield, but also hinder the construction of butanol recovery processes. In addition, *Clostridium* bacteria require strictly anaerobic conditions for their growth and butanol production. Culture under anaerobic conditions has some drawbacks, including the need for cumbersome operations such as replacing the air in the culture vessel with an inert gas such as nitrogen gas. Furthermore, the growth rate of microorganisms under anaerobic conditions is extremely slow, resulting in a low rate of butanol production accompanied by the growth of *Clostridium* bacteria.

Consequently, techniques for producing butanol using aerobic or facultative anaerobic bacteria have been explored.

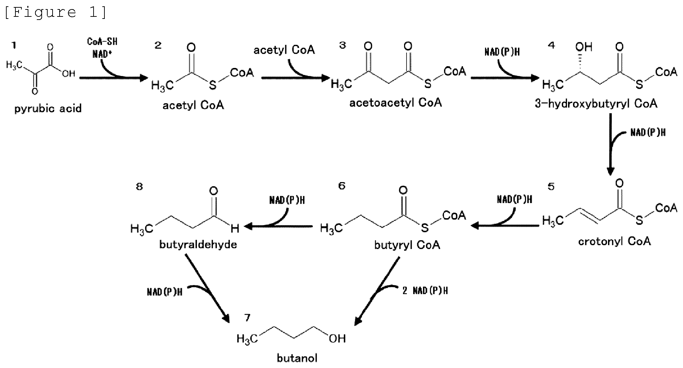

*Clostridium* bacteria have a metabolic pathway for producing butanol from glucose via pyruvate, acetyl-CoA, crotonyl-CoA, and butyryl-CoA, as shown in Fig. 1. Based on this pathway, a metabolic pathway from pyruvate to butanol via butyryl-CoA can be constructed in bacteria that do not naturally produce butanol.

According to Non Patent Literature 1, it is theoretically possible for aerobic bacteria and facultative anaerobic bacteria to produce butyryl-CoA from glucose, because the reaction from crotonyl-CoA to butyryl-CoA is encompassed in the reduction reactions of enoyl-CoA found in ubiquitous pathways such as fatty acid synthesis and fatty acid beta-oxidation pathway. However, aerobic bacteria and facultative anaerobic bacteria cannot actually produce butanol from butyryl-CoA. Therefore, various methods have been proposed for producing butanol by culturing transformants obtained by introducing, into an aerobic or facultative anaerobic bacterial host, one or more exogenous genes encoding enzymes that catalyze a metabolic pathway for producing butanol from butyryl-CoA.

For example, Patent Literature 1 discloses that butanol was produced using a transformant obtained by introducing, into *Escherichia coli,* genes for enzymes that catalyze a metabolic pathway from acetyl-CoA to butanol (acetyl-CoA acetyltransferase, β-hydroxybutyl-CoA dehydrogenase, 3-hydroxybutyryl-CoA dehydratase, butyryl-CoA dehydrogenase, butyrylaldehyde dehydrogenase, and butanol dehydrogenase).

However, the method described in Patent Literature 1 is a method for producing butanol using biomass-derived sugars as a carbon source. As described above, the use of biomass requires complex processes for converting biomass into sugars and thus is costly, and the industrial use of biomass can have adverse environmental impacts despite its intended purpose.

### Citation List

### Patent Literature

[Patent Literature 1] JP5243748B

### Non Patent Literature

[Non Patent Literature 1] Advances in Biochemical Engineering/biotechnology 155:141-163

### Summary of Invention

### Technical Problem

The objective of the present invention is to provide a transformant of a *Hydrogenophilus* bacterium, in other words, a transformed *Hydrogenophilus* bacterium, that is capable of efficiently producing butanol utilizing carbon dioxide as a sole carbon source, and a method for efficiently producing butanol using this transformant.

### Solution to Problem

The present inventor has conducted extensive investigations to solve the above-mentioned problems and has obtained the following findings.
(i) *Hydrogenophilus* bacteria, like other bacteria, can produce butyryl-CoA from pyruvate, but they lack genes encoding enzymes that catalyze a reaction for producing butanol and do not naturally produce butanol. Therefore, genes encoding enzymes that catalyze the reaction for producing butanol need to be introduced into the bacteria in order to provide them with the capability to produce butanol.
(ii) A gene encoding an enzyme having butyraldehyde dehydrogenase activity for producing butyraldehyde from butyryl-CoA (hereinafter sometimes referred to as "butyraldehyde dehydrogenase") and a gene encoding an enzyme having butanol dehydrogenase activity for producing butanol from butyraldehyde (hereinafter sometimes referred to as "butanol dehydrogenase") are expressible in *Hydrogenophilus* bacteria by introducing these genes thereinto. This makes it possible for *Hydrogenophilus* bacteria to produce butanol.

The same effect can be achieved by introducing, instead of the above two genes, a gene encoding a multifunctional enzyme having butyraldehyde dehydrogenase activity for producing butyraldehyde from butyryl-CoA and butanol dehydrogenase activity for producing butanol from butyraldehyde (hereinafter sometimes referred to as "butyraldehyde-butanol dehydrogenase").

The present invention has been completed on the basis of the above findings and provides the following [1] to [6].
[1] A transformant (transformed bacterium) obtained by introducing, into a *Hydrogenophilus* bacterium, any one of the following genes (1) to (5).
   (1) A gene encoding an enzyme having butyraldehyde dehydrogenase activity and a gene encoding an enzyme having butanol dehydrogenase activity
   (2) A gene encoding a multifunctional enzyme having butyraldehyde dehydrogenase activity and butanol dehydrogenase activity
   (3) A gene encoding an enzyme having butyraldehyde dehydrogenase activity and a gene encoding a multifunctional enzyme having butyraldehyde dehydrogenase activity and butanol dehydrogenase activity
   (4) A gene encoding an enzyme having butanol dehydrogenase activity and a gene encoding a multifunctional enzyme having butyraldehyde dehydrogenase activity and butanol dehydrogenase activity
   (5) A gene encoding an enzyme having butyraldehyde dehydrogenase activity, a gene encoding an enzyme having butanol dehydrogenase activity, and a gene encoding a multifunctional enzyme having butyraldehyde dehydrogenase activity and butanol dehydrogenase activity
[2] The transformant according to [1], wherein the following DNA (a), (b), (c), (d), or (e) is used as the gene encoding a multifunctional enzyme having butyraldehyde dehydrogenase activity and butanol dehydrogenase activity.
   (a) A DNA comprising the nucleotide sequence of SEQ ID NO: 1, 3, 5, 7, or 9
   (b) A DNA comprising a nucleotide sequence that has 90% or more identity to SEQ ID NO: 1, 3, 5, 7, or 9 and encoding a polypeptide having butyraldehyde dehydrogenase activity and butanol dehydrogenase activity
   (c) A DNA encoding a polypeptide comprising the amino acid sequence of SEQ ID NO: 2, 4, 6, 8, or 10
   (d) A DNA encoding a polypeptide comprising an amino acid sequence that has 90% or more identity to SEQ ID NO: 2, 4, 6, 8, or 10, the polypeptide having butyraldehyde dehydrogenase activity and butanol dehydrogenase activity
   (e) A DNA encoding a polypeptide comprising an amino acid sequence that has 1 to 100 amino acids deleted, substituted, inserted, or added in the amino acid sequence of SEQ ID NO: 2, 4, 6, 8, or 10, the polypeptide having butyraldehyde dehydrogenase activity and butanol dehydrogenase activity
[3] The transformant according to [1] or [2], wherein the following DNA (f), (g), (h), (i), or (j) is used as the gene encoding an enzyme having butyraldehyde dehydrogenase activity.
   (f) A DNA comprising the nucleotide sequence of SEQ ID NO: 11
   (g) A DNA comprising a nucleotide sequence that has 90% or more identity to the nucleotide sequence of SEQ ID NO: 11 and encoding a polypeptide having butyraldehyde dehydrogenase activity
   (h) A DNA encoding a polypeptide comprising the amino acid sequence of SEQ ID NO: 12
   (i) A DNA encoding a polypeptide comprising an amino acid sequence that has 90% or more identity to SEQ ID NO: 12, the polypeptide having butyraldehyde dehydrogenase activity
   (j) A DNA encoding a polypeptide comprising an amino acid sequence that has 1 to 100 amino acids deleted, substituted, inserted, or added in the amino acid sequence of SEQ ID NO: 12, the polypeptide having butyraldehyde dehydrogenase activity
[4] The transformant according to any one of [1] to [3],
   wherein the following DNA (k), (l), (m), (n), or (o) is used as the gene encoding an enzyme having butanol dehydrogenase activity.
   (k) A DNA comprising the nucleotide sequence of SEQ ID NO: 13, 15, 17, or 19
   (l) A DNA comprising a nucleotide sequence that has 90% or more identity to the nucleotide sequence of SEQ ID NO: 13, 15, 17, or 19 and encoding a polypeptide having butanol dehydrogenase activity
   (m) A DNA encoding a polypeptide comprising the amino acid sequence of SEQ ID NO: 14, 16, 18, or 20
   (n) A DNA encoding a polypeptide comprising an amino acid sequence that has 90% or more identity to SEQ ID NO: 14, 16, 18, or 20, the polypeptide having butanol dehydrogenase activity
   (o) A DNA encoding a polypeptide comprising an amino acid sequence that has 1 to 100 amino acids deleted, substituted, inserted, or added in the amino acid sequence of SEQ ID NO: 14, 16, 18, or 20, the polypeptide having butanol dehydrogenase activity
[5] The transformant according to any one of [1] to [4], wherein the *Hydrogenophilus* bacterium is *Hydrogenophilus thermoluteolus.*
[6] A method for producing butanol, comprising a step of culturing the transformant according to any one of [1] to [5] using substantially only carbon dioxide as a carbon source.

### Advantageous Effects of Invention

Measures to counter the increase in atmospheric carbon dioxide entail reduction of carbon dioxide emissions and fixation of emitted carbon dioxide. In order to reduce carbon dioxide emissions, solar, wind, geothermal, and similar energies are utilized in place of fossil energy. However, the utilization of such energies is not yet extensive enough to repress the buildup of atmospheric carbon dioxide. Consequently, there is need to enhance atmospheric carbon fixation or recycling of emitted carbon dioxide.

Carbon dioxide can be fixed through physical or chemical processes. However, fixation of carbon dioxide utilizing living cells allows the production of organic substances that can be used as food, feed, or fuel. In other words, carbon dioxide itself can be directly converted into valuable substances. Accordingly, the twin problems of global warming due to increased atmospheric carbon dioxide and scarcity of food, feed, and fuel can be solved. Further, in-demand chemical product raw materials can be produced while suppressing global warming attributed to increased carbon dioxide emissions.

Hydrogen-oxidizing bacteria that can grow by utilizing the chemical energy generated by the reaction of hydrogen with oxygen and by using carbon dioxide as a sole carbon source, can produce chemical products from a mixture of oxygen, hydrogen, and carbon dioxide gases as raw material. Therefore, the bacteria can achieve the efficient conversion of carbon dioxide into organic compounds and can be cultured in a simple culture medium. Typically, hydrogen-oxidizing bacteria grow slowly, but the growth rate of *Hydrogenophilus* bacteria is exceptionally high. The Journal of Mitsubishi Research Institute No. 34 1999 describes *Hydrogenophilus* bacteria as follows: "Their proliferative capacity is so high that their carbon dioxide fixation ability cannot be compared with that of plants, which truly indicates the high carbon dioxide fixation ability of microorganisms".

*Hydrogenophilus* bacteria originally possess genes encoding enzymes that catalyze the production of butyryl-CoA from pyruvic acid. However, they do not have genes encoding enzymes that catalyze the subsequent conversion of butyryl-CoA to butanol.

The present inventor has found that when a heterologous gene is introduced into *Hydrogenophilus* bacteria, it is difficult to express functional proteins. Nevertheless, according to the present invention, by introducing a gene encoding an enzyme having butyraldehyde dehydrogenase activity and a gene encoding an enzyme having butanol dehydrogenase activity into *Hydrogenophilus* bacteria, these genes can be expressed in the *Hydrogenophilus* bacteria. As a result, the transformed *Hydrogenophilus* bacteria are capable of producing butanol using carbon dioxide as a sole carbon source. Same results are obtained when a gene encoding a multifunctional enzyme having butyraldehyde dehydrogenase activity and butanol dehydrogenase activity is introduced into *Hydrogenophilus* bacteria, instead of introducing above two-kind genes into *Hydrogenophilus* bacteria.

As described above, *Hydrogenophilus* bacteria have an atypically remarkable carbon dioxide fixation ability among organisms having carbon dioxide fixation ability. Therefore, using the transformant of the present invention enables industrial-scale production of butanol through fixation of carbon dioxide.

### Brief Description of Drawings

[Fig. 1] Fig. 1 is a diagram illustrating a metabolic pathway for producing butanol from pyruvate, which is present in many microorganisms.
[Fig. 2] Fig. 2 is gas chromatography chromatograms showing that butyraldehyde or butanol was produced from butyryl-CoA as a substrate by the crude enzymes produced by the transformants prepared in the Examples.
[Fig. 3] Fig. 3 is gas chromatography chromatograms showing that butanol was produced from butyraldehyde as a substrate by the crude enzymes produced by the transformants prepared in the Examples.

### Embodiments of the Invention

Hereinafter, the present invention will be described in detail.

### (1) Transformant capable of producing butanol

The transformant of the present invention is a transformant obtained by introducing, into a host *Hydrogenophilus* bacterium, any one of the following genes (1) to (5). In other words, the transformant of the present invention has an exogenous gene that is any one of the following genes (1) to (5).
(1) A gene encoding an enzyme having butyraldehyde dehydrogenase activity and a gene encoding an enzyme having butanol dehydrogenase activity
(2) A gene encoding a multifunctional enzyme having butyraldehyde dehydrogenase activity and butanol dehydrogenase activity
(3) A gene encoding an enzyme having butyraldehyde dehydrogenase activity and a gene encoding a multifunctional enzyme having butyraldehyde dehydrogenase activity and butanol dehydrogenase activity
(4) A gene encoding an enzyme having butanol dehydrogenase activity and a gene encoding a multifunctional enzyme having butyraldehyde dehydrogenase activity and butanol dehydrogenase activity
(5) A gene encoding an enzyme having butyraldehyde dehydrogenase activity, a gene encoding an enzyme having butanol dehydrogenase activity, and a gene encoding a multifunctional enzyme having butyraldehyde dehydrogenase activity and butanol dehydrogenase activity

The butyraldehyde-butanol dehydrogenase gene, the butyraldehyde dehydrogenase gene, and the butanol dehydrogenase gene may each be DNA isolated from naturally occurring bacteria or may be DNA artificially synthesized using methods known to those skilled in the art.

The butyraldehyde-butanol dehydrogenase gene can be introduced in one type or in any combination of two or more types. The butyraldehyde dehydrogenase gene can be introduced in one type or in any combination of two or more types. The butanol dehydrogenase gene can be introduced in one type or in any combination of two or more types. The present invention also encompasses a transformant obtained by introducing not only any one of the above genes (1) to (5), but also another gene.

### Butyraldehyde-butanol dehydrogenase gene

The gene encoding butyraldehyde-butanol dehydrogenase does not need to have been previously identified as a gene of butyraldehyde-butanol dehydrogenase and may be any DNA encoding a multifunctional enzyme having butyraldehyde dehydrogenase activity for producing butyraldehyde from butyryl-CoA and butanol dehydrogenase activity for producing butanol from butyraldehyde. This multifunctional enzyme may be a bifunctional enzyme having only butyraldehyde dehydrogenase activity and butanol dehydrogenase activity.

Examples of the butyraldehyde-butanol dehydrogenase gene that can be used in the present invention include those shown in Table 1. Table 1 shows the bacterial species from which the enzyme originates, the nucleotide sequences of the gene encoding the enzyme, and the amino acid sequences of the enzyme.

**[Table 1]**

| Origin of butyraldehyde-butanol dehydrogenase gene | Nucleotide sequence | Amino acid sequence |
|---|---|---|
| *Thermoanaerobacter mathranii* | SEQ ID NO: 1 | SEQ ID NO: 2 |
| *Fonticella tunisiensis* | SEQ ID NO: 3 | SEQ ID NO: 4 |
| *Thermoclostridium caenicola* | SEQ ID NO: 5 | SEQ ID NO: 6 |
| *Thermoanaerobacter ethanolicus* | SEQ ID NO: 7 | SEQ ID NO: 8 |
| *Bacillus coagulans* | SEQ ID NO: 9 | SEQ ID NO: 10 |

Each butyraldehyde-butanol dehydrogenase shown in Table 1 is a bifunctional enzyme having both butyraldehyde dehydrogenase activity and butanol dehydrogenase activity.

In the present invention, a DNA comprising the nucleotide sequence of SEQ ID NO: 1, 3, 5, 7, or 9 (in particular, a DNA consisting of SEQ ID NO: 1, 3, 5, 7, or 9) can be used.

A DNA comprising a nucleotide sequence that has 90% or more, even 95% or more, even 98% or more, even 99% or more identity to SEQ ID NO: 1, 3, 5, 7, or 9 (in particular, a DNA consisting of a nucleotide sequence that has 90% or more, even 95% or more, even 98% or more, even 99% or more identity to SEQ ID NO: 1, 3, 5, 7, or 9) and encoding a polypeptide having butyraldehyde dehydrogenase activity and butanol dehydrogenase activity can also be used.

In addition, a DNA encoding a polypeptide comprising the amino acid sequence of SEQ ID NO: 2, 4, 6, 8, or 10 (in particular, a polypeptide consisting of the amino acid sequence of SEQ ID NO: 2, 4, 6, 8, or 10) can be used.

A DNA encoding a polypeptide comprising an amino acid sequence that has 90% or more, even 95% or more, even 98% or more, even 99% or more identity to SEQ ID NO: 2, 4, 6, 8, or 10 (in particular, a polypeptide consisting of an amino acid sequence that has 90% or more, even 95% or more, even 98% or more, even 99% or more identity to SEQ ID NO: 2, 4, 6, 8, or 10) and having butyraldehyde dehydrogenase activity and butanol dehydrogenase activity can also be used.

Furthermore, a DNA encoding a polypeptide comprising an amino acid sequence that has 1 to 100, 1 to 50, 1 to 30, 1 to 10, 1 to 5, 1 to 4, 1 to 3, or 1 to 2 amino acids, or 1 amino acid deleted, substituted, inserted, or added in the amino acid sequence of SEQ ID NO: 2, 4, 6, 8, or 10 (in particular, a polypeptide consisting of an amino acid sequence that has 1 to 100, 1 to 50, 1 to 30, 1 to 10, 1 to 5, 1 to 4, 1 to 3, or 1 to 2 amino acids, or 1 amino acid deleted, substituted, inserted, or added in the amino acid sequence of SEQ ID NO: 2, 4, 6, 8, or 10) and having butyraldehyde dehydrogenase activity and butanol dehydrogenase activity can be used.

The functionality of a bifunctional enzyme having the amino acid sequence of SEQ ID NO: 2, 4, 6, 8, or 10 in *Hydrogenophilus* bacteria has been confirmed, as demonstrated in the Examples section, by the fact that the crude enzyme produced by the bacterial cells produced butanol from butyryl-CoA as a substrate in the presence of NADH.

In the present invention, the presence of butyraldehyde dehydrogenase activity using butyryl-CoA as a substrate and butanol dehydrogenase activity using butyraldehyde as a substrate in a polypeptide of interest is determined by reacting the polypeptide with butyryl-CoA in the presence of NADH and detecting the resulting butanol with a gas chromatograph or the like.

### Butyraldehyde dehydrogenase gene

The gene encoding an enzyme having butyraldehyde dehydrogenase activity does not need to have been previously identified as a gene of butyraldehyde dehydrogenase and may be any DNA encoding a polypeptide having butyraldehyde dehydrogenase activity.

An example of the butyraldehyde dehydrogenase gene that can be used in the present invention is the butyraldehyde dehydrogenase gene of *Thermus thermophilus* consisting of the nucleotide sequence of SEQ ID NO: 11. The butyraldehyde dehydrogenase of *Thermus thermophilus* is not a multifunctional enzyme having alcohol dehydrogenase.

In the present invention, a DNA comprising the nucleotide sequence of SEQ ID NO: 11 (in particular, a DNA consisting of the nucleotide sequence of SEQ ID NO: 11) can be used.

A DNA comprising a nucleotide sequence that has 90% or more, even 95% or more, even 98% or more, even 99% or more identity to SEQ ID NO: 11 (in particular, a DNA consisting of a nucleotide sequence that has 90% or more, even 95% or more, even 98% or more, even 99% or more identity to SEQ ID NO: 11) and encoding a polypeptide having butyraldehyde dehydrogenase activity can also be used.

Another example of the butyraldehyde dehydrogenase gene is a DNA encoding the butyraldehyde dehydrogenase of *Thermus thermophilus* consisting of the amino acid sequence of SEQ ID NO: 12. In the present invention, a DNA encoding a polypeptide comprising the amino acid sequence of SEQ ID NO: 12 (in particular, a polypeptide consisting of the amino acid sequence of SEQ ID NO: 12) can be used.

A DNA encoding a polypeptide comprising an amino acid sequence that has 90% or more, even 95% or more, even 98% or more, even 99% or more identity to SEQ ID NO: 12 (in particular, a polypeptide consisting of an amino acid sequence that has 90% or more, even 95% or more, even 98% or more, even 99% or more identity to SEQ ID NO: 12) and having butyraldehyde dehydrogenase activity can also be used.

Furthermore, a DNA encoding a polypeptide comprising an amino acid sequence that has 1 to 100, 1 to 50, 1 to 30, 1 to 10, 1 to 5, 1 to 4, 1 to 3, or 1 to 2 amino acids, or 1 amino acid deleted, substituted, inserted, or added in the amino acid sequence of SEQ ID NO: 12 (in particular, a polypeptide consisting of an amino acid sequence that has 1 to 100, 1 to 50, 1 to 30, 1 to 10, 1 to 5, 1 to 4, 1 to 3, or 1 to 2 amino acids, or 1 amino acid deleted, substituted, inserted, or added in the amino acid sequence of SEQ ID NO: 12) and having butyraldehyde dehydrogenase activity can be used.

In the present invention, a gene of aldehyde-alcohol dehydrogenase which can produce butyraldehyde from butyryl-CoA by its aldehyde dehydrogenase activity, but cannot produce butanol from butyraldehyde by its alcohol dehydrogenase activity can also be used as the butyraldehyde dehydrogenase gene. This enzyme is generally referred to as aldehyde-alcohol dehydrogenase, but in the present invention, the enzyme is classified as butyraldehyde dehydrogenase.

In the present invention, the presence of butyraldehyde dehydrogenase activity using butyryl-CoA as a substrate in a polypeptide of interest is determined by reacting the polypeptide with butyryl-CoA in the presence of NADH and detecting the resulting butyraldehyde with a gas chromatograph or the like.

### Butanol dehydrogenase gene

The gene encoding an enzyme having butanol dehydrogenase activity does not need to have been previously identified as a gene of butanol dehydrogenase and may be any DNA encoding a polypeptide having butanol dehydrogenase activity for producing butanol from butyraldehyde.

Examples of the butanol dehydrogenase gene that can be used in the present invention include those shown in Table 2. Table 2 shows the bacterial species from which the enzyme originates, the nucleotide sequences of the gene encoding the enzyme, and the amino acid sequences of the enzyme.

**[Table 2]**

| Origin of butanol dehydrogenase gene | Nucleotide sequence | Amino acid sequence |
|---|---|---|
| *Bacillus coagulans* | SEQ ID NO: 13 | SEQ ID NO: 14 |
| *Zymomonas mobilis* subsp. *mobilis* | SEQ ID NO: 15 | SEQ ID NO: 16 |
| *Thermoanaerobacter thermocopriae* | SEQ ID NO: 17 | SEQ ID NO: 18 |
| *Geobacillus stearothermophilus* | SEQ ID NO: 19 | SEQ ID NO: 20 |

Neither the butanol dehydrogenase of *Bacillus coagulans* consisting of the amino acid sequence of SEQ ID NO: 14 nor the butanol dehydrogenase of *Zymomonas mobilis* subsp. *mobilis* consisting of the amino acid sequence of SEQ ID NO: 16 is a multifunctional enzyme having aldehyde dehydrogenase.

The butanol dehydrogenase of *Thermoanaerobacter thermocopriae* consisting of the amino acid sequence of SEQ ID NO: 18 and the butanol dehydrogenase of *Geobacillus stearothermophilus* consisting of the amino acid sequence of SEQ ID NO: 20 belong to aldehyde-alcohol dehydrogenases, but neither of them can produce butyraldehyde from butyryl-CoA.

In the present invention, a DNA comprising a nucleotide sequence that has 90% or more, even 95% or more, even 98% or more, even 99% or more identity to SEQ ID NO: 13, 15, 17, or 19 (in particular, a DNA consisting of a nucleotide sequence that has 90% or more, even 95% or more, even 98% or more, even 99% or more identity to SEQ ID NO: 13, 15, 17, or 19) and encoding a polypeptide having butanol dehydrogenase activity can also be used.

In addition, a DNA encoding a polypeptide comprising an amino acid sequence that has 90% or more, even 95% or more, even 98% or more, even 99% or more identity to the amino acid sequence of SEQ ID NO: 14, 16, 18, or 20 (in particular, a polypeptide consisting of an amino acid sequence that has 90% or more, even 95% or more, even 98% or more, even 99% or more identity to the amino acid sequence of SEQ ID NO: 14, 16, 18, or 20) and having butanol dehydrogenase activity can be used.

Furthermore, a DNA encoding a polypeptide comprising an amino acid sequence that has 1 to 100, 1 to 50, 1 to 30, 1 to 10, 1 to 5, 1 to 4, 1 to 3, or 1 to 2 amino acids, or 1 amino acid deleted, substituted, inserted, or added in the amino acid sequence of SEQ ID NO: 14, 16, 18, or 20 (in particular, a polypeptide consisting of an amino acid sequence that has 1 to 100, 1 to 50, 1 to 30, 1 to 10, 1 to 5, 1 to 4, 1 to 3, or 1 to 2 amino acids, or 1 amino acid deleted, substituted, inserted, or added in the amino acid sequence of SEQ ID NO: 14, 16, 18, or 20) and having butanol dehydrogenase activity can be used.

As described above, in the present invention, a gene of aldehyde-alcohol dehydrogenase which can produce butanol from butyraldehyde by its alcohol dehydrogenase activity, but cannot produce butyraldehyde from butyryl-CoA by its aldehyde dehydrogenase activity can also be used as the gene of an enzyme having butanol dehydrogenase activity. This enzyme is generally referred to as aldehyde-alcohol dehydrogenase, but in the present invention, the enzyme is classified as butanol dehydrogenase.

In the present invention, the presence of butanol dehydrogenase activity using butyraldehyde as a substrate in a polypeptide of interest is determined by reacting the polypeptide with butyraldehyde in the presence of NADH and detecting the resulting butanol with a gas chromatograph or the like.

In view of codon degeneracy or codon preference in *Hydrogenophilus* bacteria, the DNA sequence encoding a protein comprising the amino acid sequence of SEQ ID NO: 2, 4, 6, 8, 10, 12, 14, 16, 18, or 20 may be subjected to a variety of nucleotide substitutions in the coding region, as long as such substitutions do not alter the amino acid sequence of the protein expressed from the coding region.

In the present invention, the identities of nucleotide sequences and amino acid sequences were calculated using GENETYX ver. 17 (GENETYX).

In the present invention, a DNA comprising a nucleotide sequence that has 90% or more but less than 100% identity to the nucleotide sequence of a given DNA and encoding a polypeptide having the same type of activity as the polypeptide encoded by the given DNA is called a homolog of the given DNA. A DNA encoding a polypeptide comprising an amino acid sequence that has 90% or more but less than 100% identity to the amino acid sequence of the polypeptide encoded by a given DNA and having the same type of activity as the polypeptide encoded by the given DNA is also called a homolog of the given DNA. A DNA encoding a polypeptide comprising an amino acid sequence that has 1 to 100 amino acids deleted, substituted, inserted, or added in the amino acid sequence of the polypeptide encoded by a given DNA and having the same type of activity as the polypeptide encoded by the given DNA is also called a homolog of the given DNA.

In the present invention, a polypeptide comprising an amino acid sequence that has 90% or more but less than 100% identity to the amino acid sequence of a given polypeptide and having the same type of activity as the given polypeptide is called a homolog of the given polypeptide. A polypeptide comprising an amino acid sequence that has 1 to 100 amino acids deleted, substituted, inserted, or added in the amino acid sequence of a given polypeptide and having the same type of activity as the given polypeptide is also called a homolog of the given polypeptide.

### Hydrogenophilus bacteria

Examples of *Hydrogenophilus* bacteria include *Hydrogenophilus thermoluteolus, Hydrogenophilus halorhabdus, Hydrogenophilus denitrificans, Hydrogenophilus hirschii, Hydrogenophilus islandicus, Hydrogenophilus thiooxidans, Hydrogenophilus* bacterium strain Mar3 *(Hydrogenophilus* sp. Mar3), and *Hydrogenophilus* bacterium strain Z1038 *(Hydrogenophilus* sp. Z1038). In particular, *Hydrogenophilus thermoluteolus* is preferable because its superior growth rate enables top-level carbon dioxide fixation among carbon dioxide fixing microorganisms.

*Hydrogenophilus* bacteria have been easily isolated from diverse regions everywhere on the earth. A preferable strain of *Hydrogenophilus thermoluteolus* is strain TH-1 (NBRC 14978). *Hydrogenophilus thermoluteolus* strain TH-1 (NBRC 14978) exhibits the highest rapid growth rate among carbon dioxide fixing microorganisms (Agricultural and Biological Chemistry, 41, 685-690 (1977)) (It proliferates double per hour.). *Hydrogenophilus thermoluteolus* strain NBRC 14978 is internationally deposited under the Budapest Treaty, and is thus publicly available.

The *Hydrogenophilus* bacteria as hosts is to be introduced in the present invention may be bacteria isolated from nature or genetically modified bacteria derived from bacteria isolated from nature. Genetic modification may be intended, for example, to allow high expression of an introduced gene.

Such a genetic modification can be made, for example, by removing (curing) endogenous plasmids in *Hydrogenophilus* bacteria. Methods for removing endogenous plasmids are well known and include, for example, treating with chemicals such as novobiocin, SDS, acriflavine, and ethidium bromide; introducing a plasmid having the same origin of replication as those of endogenous plasmids to destabilize the endogenous plasmids; and utilizing the phenomenon of plasmid incompatibility, such as disrupting factors involved in the plasmid partition system to destabilize endogenous plasmids.

### Methods for producing transformants

Next, methods for obtaining transformants by introducing genes of above mentioned (1) to (5) into *Hydrogenophilus* bacteria are described.

The introduction of these genes into *Hydrogenophilus* bacteria can be carried out using common methods for introducing exogenous genes into bacteria. These genes may be directly introduced into *Hydrogenophilus* bacteria. Alternatively, they may be incorporated into a vector for transformation such as a plasmid vector, viral vector, cosmid, fosmid, BAC, or YAC, and then, an obtained vector may be introduced into *Hydrogenophilus* bacteria.

Vectors for transformation should contain a DNA which controls the autonomous replication function within *Hydrogenophilus* bacteria, and examples include broad-host-range vectors pRK415 (GenBank: EF437940.1), pBHR1 (GenBank: Y14439.1), pMMB67EH (ATCC 37622), pCAR1 (NCBI Reference Sequence: NC_004444.1), pC194 (NCBI Reference Sequence: NC_002013.1), pK18mobsacB (GenBank: FJ437239.1), pUB110 (NCBI Reference Sequence: NC_001384.1), vectors obtained by genetically modifying these vectors (For example, pCAMO-6), and the like.

Among them, pCAMO-6 is preferable. Those skilled in the art can prepare pCAMO-6 according to the Examples of this specification.

Examples of promoters in vectors include tac promoter, lac promoter, trc promoter, or each of promoters OXB1 and OXB11 to OXB20 from Oxford Genetics Ltd. Examples of terminators in vectors include the T1T2 terminator of *Escherichia coli* rRNA operon rrnB, the t0 transcription terminator of bacteriophage λ, T7 terminator, and the like.

Introduction of these genes into a *Hydrogenophilus* bacterium (transformation) can be carried out by publicly known methods such as calcium chloride method, rubidium chloride method, and electric pulse method (electroporation method).

### (2) Method for producing butanol

The present invention provides a method for producing butanol with use of the transformant of the present invention described above. The method includes a step of culturing the transformant of the present invention using an inorganic or organic culture medium while supplying a gas containing carbon dioxide, preferably, a mixture of gases containing hydrogen, oxygen, and carbon dioxide. The supplied gas is preferably a mixture of gases consisting of hydrogen, oxygen, and carbon dioxide. However, different kinds of gases can be mixed within, to the extent that butanol can be produced efficiently.

*Hydrogenophilus* bacteria can grow using hydrogen as a source of energy and using carbon dioxide as a sole carbon source, and thus, carbon dioxide can be fixed efficiently particularly by producing butanol by using substantially only carbon dioxide (in particular, by using only carbon dioxide) as a carbon source. Therefore, using an inorganic culture medium that does not contain carbon sources such as organic substances and carbonates is preferable. Namely, carrying out culture using substantially only carbon dioxide (in particular, using only carbon dioxide) as a carbon source is preferable. "Using substantially only carbon dioxide as a carbon source" encompass cases in which an unavoidable amount of other carbon sources is mixed within.

The pH of the culture medium is preferably 6.2 to 8, more preferably 6.4 to 7.4, and furthermore preferably 6.6 to 7. When the pH is within this range, bacteria grow well and mixed gases dissolves well into the culture medium, and butanol can be produced efficiently.

When batch culture is utilized, mixed gases can be entrapped within an airtight culture container and static culture or shaking culture can be carried out. Shaking culture is preferable in that better dissolution of mixed gases into the culture medium can be achieved. When continuous culture is utilized, mixed gases can be continuously supplied into an airtight culture container and shaking culture can be carried out, or the transformant can be cultured using an airtight culture container while introducing mixed gases into the culture medium by bubbling.

The volume ratio of hydrogen, oxygen, and carbon dioxide (hydrogen: oxygen: carbon dioxide) in the supplied gas mixture is preferably 1.75 to 7.5:1:0.25 to 3, more preferably 5 to 7.5:1:1 to 2, and even more preferably 6.25 to 7.5:1:1.5. When the volume ratio is within this range, bacteria grow well, and butanol can be produced efficiently.

The supply rate of mixed gases or raw material gases can be 10 to 60 L/hour, in particular 10 to 40 L/hour, in particular 10 to 20 L/hour, per 1 L of culture medium. When the supply rate is within this range, transformants grow well and butanol can be produced efficiently, and the amount of wasted mixed gases can be reduced.

The culture temperature is preferably 35 to 55°C, more preferably 37 to 52°C, and even more preferably 50 to 52°C. When the temperature is within this range, transformants grow well, and butanol can be produced efficiently.

Butanol is produced in the medium solution by culturing the transformant in the above-described manner. Collecting the medium solution will enable the recovery of butanol, however, butanol can furthermore be separated from the medium solution by publicly known methods. Such publicly known methods include membrane separation, distillation, and the like.

### Examples

The present invention will now be described with reference to examples. The technical scope of the present invention is not limited by the following examples.

### (1) Construction of plasmid vector (pCAMO-6)

The method for constructing a pCAMO-6 plasmid vector used for the introduction of aldehyde dehydrogenase gene and alcohol dehydrogenase gene is described below.

### (1-1) Preparation of tac promoter-containing DNA fragment

A pMAL-c5X plasmid manufactured by New England Biolabs was used as a template to amplify a DNA fragment containing a tac promoter by PCR using a pair of primers shown below. Unless otherwise noted, PCR described below was performed in the usual manner using 2720 Thermal Cycler manufactured by Thermo Fisher Scientific Inc. and using KOD FX Neo (manufactured by Toyobo Co., Ltd.) as a reaction reagent.

### Primers for amplification of tac promoter

(a-1) 5'-TTTTATAACCCGGGCCATCGACTGCACGGTGCACC-3' (SEQ ID NO: 21)
(b-1) 5'-TGCTAGCACTGTTTCCTGTGTGAAATTGTTATCCG-3' (SEQ ID NO: 22)

The primer (a-1) contains a SmaI restriction enzyme site as indicated by underlining.

The resulting reaction mixture was subjected to electrophoresis on an agarose gel. The results showed that an about 0.3-kbp DNA fragment corresponding to the tac promoter was detected. A gel portion containing the DNA fragment was cut out of the agarose gel, and the gel portion was freeze-thawed to recover the DNA fragment.

### (1-2) Preparation of multicloning site-containing DNA fragment

For the preparation of a DNA fragment containing a multicloning site, the former and latter halves of the multicloning site were separately produced by PCR using pairs of primers shown below. This method does not use any template DNA and allows each pair of primers to anneal together in their 3' terminal regions and elongate to form a double-stranded DNA.

### Primers for preparation of former half of multicloning site

(a-2) 5'-GGAAACAGTGCTAGCAGATCTGGAGGAGAAAGGCATAT-3' (SEQ ID NO: 23)
(b-2)

5′-
CAGTGCGGCCGCAAGCTTGTCGACGGAGCTCGAATTCGGATCCGATATCAGCATATGCGTTTC
TCCTCCAGA-3′ (SEQ ID NO: 24)

The 3' terminal 20-nucleotide sequences of the primers (a-2) and (b-2) are complementary to each other.

### Primers for preparation of latter half of multicloning site

(a-3) 
5′-
ACAAGCTTGCGGCCGCACTGCAGCACCATCACCACCATCATTGATAAGATCCGGCTGCTAACA
AAGCCCGAAAGGAAGCT-3′ (SEQ ID NO: 25)
(b-3)

5′-
TATTTGAATCGAGTTATTGCTCAGCGGTGGCAGCAGCCAACTCAGCTTCCTTTCGGGCTTTGT
-3′ (SEQ ID NO: 26)

The 3' terminal 20-nucleotide sequences of the primers (a-3) and (b-3) are complementary to each other.

The resulting reaction mixture was subjected to electrophoresis on an agarose gel. The results showed that about 0.1-kbp DNA fragments corresponding to the former and latter halves of the multicloning site were detected. Gel portions containing the DNA fragments were cut out of the agarose gel, and the gel portions were freeze-thawed to recover the DNA fragments.

Overlap extension PCR was performed through use of the recovered DNA fragments corresponding to the former and latter halves of the multicloning site as a template. The 5' terminal 20-nucleotide sequences of the primers (b-2) and (a-3) used to amplify these template DNA fragments are complementary to each other. For the overlap extension PCR, a combination of the primers (a-2) and (b-3) shown above was used to construct a DNA containing the multicloning site.

The resulting reaction mixture was subjected to electrophoresis on an agarose gel. The results showed that an about 0.2-kbp DNA fragment corresponding to the multicloning site was detected. The DNA fragment was recovered from the gel.

### (1-3) Preparation of DNA fragment containing rrnB terminator joined to origin of replication of pUC19

Plasmid pMAL-c5X manufactured by New England Biolabs was used as a template to amplify a DNA fragment containing rrnB terminator by PCR using a pair of primers shown below.

### Primers for amplification of rrnB terminator

(a-4) 5'-TAACTCGATTCAAATAAAACGAAAGGCTCAGTCGA-3' (SEQ ID NO: 27)
(b-4) 5'-CCTAGATCCGCGGAGTTTGTAGAAACGCAAAAAGG-3' (SEQ ID NO: 28)

In addition, pUC19 plasmid was used as a template to amplify a DNA fragment containing an origin of DNA replication by PCR using a pair of primers shown below.

### Primers for amplification of origin of DNA replication of pUC19

(a-5) 5'-ACAAACTCCGCGGATCTAGGTGAAGATCCTTTTTG-3' (SEQ ID NO: 29)
(b-5) 5'-CGTCCGCGGCCAGCAAAAGGCCAGGAACCGTAAAA-3' (SEQ ID NO: 30)

Each resulting reaction mixture was subjected to electrophoresis on an agarose gel. The results showed that an about 0.3-kbp DNA fragment for the rrnB terminator and an about 0.8-kbp DNA fragment for the origin of DNA replication of pUC19 were detected. Gel portions containing the DNA fragments were cut out of the agarose gel, and the gel portions were freeze-thawed to recover the DNA fragments.

Overlap extension PCR was performed through use of the recovered DNA fragments corresponding to the rrnB terminator and the origin of DNA replication of pUC19 were used as templates. The 5' terminal 20-nucleotide sequences of the primers (b-4) and (a-5) used to amplify these template DNA fragments are complementary to each other. For the overlap extension PCR, a combination of the primers (a-4) and (b-5) was used to construct a DNA fragment containing the rrnB terminator joined to the origin of replication of pUC19.

The resulting reaction mixture was subjected to electrophoresis on an agarose gel. The results showed that an about 1.0-kbp DNA fragment corresponding to a DNA containing the rrnB terminator joined to the origin of replication of pUC19 was detected. The DNA fragment was recovered from the gel

### (1-4) Preparation of DNA fragment containing neomycin/kanamycin resistance gene

Plasmid pK18mobsacB (GenBank: FJ437239.1) (Gene, 145, 69-73 (1994)), which contains a neomycin/kanamycin resistance gene (hereinafter sometimes referred to as "nptII") sequence, was used as a template for PCR. For the amplification of a DNA fragment containing the nptII gene sequence, a pair of primers shown below was used for the PCR.

### Primers for amplification of nptII gene

(a-6) 5'-TTGCTGGCCGCGGACGTAGAAAGCCTGTCCGCAGA-3' (SEQ ID NO: 31)
(b-6) 5'-GGCCCGGGTTATAAAAGCCAGTCATTAGGCCTATC-3' (SEQ ID NO: 32)

The primer (b-6) contains a SmaI restriction enzyme site as indicated by underlining.

The resulting reaction mixture was subjected to electrophoresis on an agarose gel. The results showed that an about 1.0-kbp DNA fragment corresponding to the nptII gene was detected. The DNA fragment was recovered from the gel.

### (1-5) Construction of circular plasmid

For the DNA fragments prepared in the above (1-1) to (1-4), the terminal region of the DNA fragment in the above (1-1) has a 16-bp sequence homologous to the terminal regions of the DNA fragments in the above (1-4) and (1-2); the terminal region of the DNA fragment in the above (1-2) has a 16-bp sequence homologous to the terminal regions of the DNA fragments in the above (1-1) and (1-3); the terminal region of the DNA fragment in the above (1-3) has a 16-bp sequence homologous to the terminal regions of the DNA fragments in the above (1-2) and (1-4); and the terminal region of the DNA fragment in the above (1-4) has a 16-bp sequence homologous to the terminal regions of the DNA fragments in the above (1-3) and (1-1).

Thus, the DNA fragments in the above (1-1), (1-2), (1-3), and (1-4) can be joined together into the form of a circular DNA by Gibson Assembly (Gibson et al., Nature Methods 6(5):343-345), which is one of DNA ligation methods not using restriction enzymes nor ligases. Gibson Assembly was performed using Gibson Assembly Master Mix (manufactured by New England Biolabs) to join the DNA fragments prepared in the above (1-1) to (1-4) into a circular DNA. With the obtained reaction mixture, *Escherichia coli* JM109 was transformed according to calcium chloride method, and applied to a solid LB medium containing 50 µg/mL kanamycin. The grown strain on the medium was cultured in liquid medium in the usual manner. Plasmid DNA was extracted from the culture liquid and reacted with restriction enzyme, SmaI. This reaction mixture was subjected to electrophoresis on an agarose gel. The results showed that an about 2.3-kbp DNA fragment was observed, which corresponds in size to a single piece of DNA formed by joining the DNA fragments prepared in the above (1-1) to (1-4), demonstrating successful assembly of the DNA fragments.

This circular plasmid DNA, which is replicable in *Escherichia coli,* was named pCAMO-1.

### (1-6) Construction of plasmid vector (pCAMO-4)

*Hydrogenophilus thermoluteolus* TH-1 strain was inoculated with a platinum loop into 5 mL of liquid A medium [3.0 g of (NH₄)₂SO₄, 1.0 g of KH₂PO₄, 2.0 g of K₂HPO₄, 0.25 g of NaCl, 0.014 g of FeSO₄ · 7H₂O, 0.5 g of MgSO₄ · 7H₂O, 0.03 g of CaCl₂, 4.0 mg of MoO₃, 28 mg of ZnSO₄· 7H₂O, 2.0 mg of CuSO₄·5H₂O, 4.0 mg of H₃BO₃, 4.0 mg of MnSO₄·5H₂O, and 4.0 mg of CoCl₂·6H₂O were dissolved in 1 L of distilled water (pH 7.0)]in a test tube. The test tube was filled with a gas mixture of H₂:O₂:CO₂ = 7.5:1:1.5, and the strain was cultured at 50°C.

Endogenous plasmid, pTH1 (Microbiol Resour Announc. 2018 Aug 16; 7(6)), was prepared from the culture liquid according to the conventional alkaline-SDS method.

The prepared pTH-1 of about 66 kbp in size was cut with restriction enzymes ScaI and PvuII, and the pCAMO-1 plasmid was cut with restriction enzyme SmaI. Both of them were ligated together by T4 DNA (manufactured by Takara Bio Inc.).

With the resulting ligation reaction liquid, *Hydrogenophilus thermoluteolus* TH-1 strain (NBRC 14978) was transformed according to electric pulse method (electroporation), applied to solid A medium [3.0 g of (NH₄)₂SO₄, 1.0 g of KH₂PO₄, 2.0 g of K₂HPO₄, 0.25 g of NaCl, 0.014 g of FeSO₄·7H₂O, 0.5 g of MgSO₄·7H₂O, 0.03 g of CaCl₂, 4.0 mg of MoO₃, 28 mg of ZnSO₄· 7H₂O, 2.0 mg of CuSO₄·5H₂O, 4.0 mg of H₃BO₃, 4.0 mg of MnSO₄·5H₂O, 4.0 mg of CoCl₂·6H₂O, and 15 g of agar were dissolved in 1 L of distilled water (pH 7.0)] containing 50 µg/mL kanamycin, and cultured in a chamber filled with a gas mixture of H₂:O₂:CO₂ = 7.5:1:1.5 at 50°C for 60 hours.

Each of seven strains grown on the solid A medium was inoculated with a platinum loop into 5 mL of liquid A medium containing 50 µg/mL kanamycin in a test tube. The test tubes were filled with a gas mixture of H₂:O₂:CO₂ = 7.5:1:1.5, and the strains were cultured with shaking at 52°C. Plasmid DNA was extracted from the culture and subjected to electrophoresis on an agarose gel. The results showed that all of the 7 strains had the same plasmid DNA of about 5.5 kbp in size.

The extracted plasmid was used as a template to amplify a DNA fragment containing the endogenous plasmid pTH1 inserted in the SmaI restriction enzyme site of pCAMO-1 by PCR using a pair of primers shown below. The pair of primers correspond to the regions at both sides of the SmaI restriction enzyme site of pCAMO-1.

### Primers for amplification of inserted pTH1 DNA fragment

(a-7) 5'-AATTGTCAGATAGGCCTAATGACTGGCTTTTATAA-3' (SEQ ID NO: 33)
(b-7) 5'-TGACGCCAGAAGCATTGGTGCACCGTGCAGTCGAT-3' (SEQ ID NO: 34)

The resulting reaction mixture was subjected to electrophoresis on a 1% agarose gel. The results showed that an about 3.2-kbp DNA fragment was detected. That is, the obtained plasmid contains an about 3.2-kbp DNA fragment corresponding to a part of pTH1, which has been inserted into the SmaI restriction enzyme site of the pCAMO-1 plasmid. This about 3.2-kbp DNA fragment contains the origin of replication that functions in *Hydrogenophilus thermoluteolus* cells, so that the obtained plasmid replicates in *Hydrogenophilus thermoluteolus* cells.

The constructed plasmid was named pCAMO-4.

### (1-7) Construction of plasmid vector (pCAMO-5)

Equal moles of a pair of oligonucleotides shown below were mixed, and the mixture was gently cooled from 98°C to 20°C, thus yielding a double-stranded DNA in which the oligonucleotides were annealed. Both ends of this DNA fragment are equivalent to the protruding ends generated by cleavage with restriction enzymes BglII and NdeI. This DNA fragment and a digested DNA fragment prepared by cutting pCAMO-4 with restriction enzymes BglII and NdeI were ligated together by T4 DNA ligase.
(a-8) 5'-GATCTGGAGGAGAAACGCA-3' (SEQ ID NO: 35)
(b-8) 5'-TATGCGTTTCTCCTCCA-3' (SEQ ID NO: 36)

The constructed plasmid was named pCAMO-5.

### (1-8) Construction of plasmid vector (pCAMO-6)

The endogenous plasmid pTH-1 was used as a template to amplify a DNA fragment containing a mazF gene encoding a plasmid stabilization factor by PCR using a pair of primers shown below.

### Primers for amplification of mazF gene

(a-9) 5'-GAGGCCATCTAGGCCATGAGTAAGTCTGACGGAAC-3' (SEQ ID NO: 37)
(b-9) 5'-ATCCGGCACCCATATCTGAACCGGACGCAAACCCG-3' (SEQ ID NO: 38)

The endogenous plasmid pTH-1 was used as a template to amplify a DNA fragment containing a mazE gene encoding a plasmid stabilization factor by PCR using a pair of primers shown below.

### Primers for amplification of mazE gene

(a-10) 5'-TTCAGATATGGGTGCCGGATACCCGCCGCCCGGGC-3' (SEQ ID NO: 39)
(b-10) 5'-AAGGCCTTCATGGCCTTATTTCGCGATTCCCAAGA-3' (SEQ ID NO: 40)

The 3' terminal region of the mazF-containing DNA fragment has a 20-bp sequence homologous to the 5' terminal region of the mazE-containing DNA fragment. Thus, the mazF-containing DNA fragment and the mazE-containing DNA fragment can be joined by overlap extension PCR. The DNA fragments of mazF and mazE prepared above were mixed, and for the amplification of the joined DNA fragments, overlap extension PCR was conducted using primers (a-9) and (b-10).

The resulting reaction mixture was subjected to electrophoresis on an agarose gel. The results showed that an about 0.7-kbp DNA fragment corresponding to a DNA formed by joining the mazF-containing DNA fragment and the mazE-containing DNA fragment was detected. The DNA fragment was recovered from the gel.

The prepared DNA fragment of about 0.7 kbp in size was cut with restriction enzyme SfiI, and the pCAMO-5 plasmid was cut with restriction enzyme SfiI. Both fragments were ligated together by T4 DNA ligase.

The constructed plasmid was named pCAMO-6.

### (2) Construction of gene expression plasmids

Gene expression plasmids were constructed by Gibson Assembly.

DNA fragments consisting of the genes from different bacteria shown below were amplified by PCR.

### Butyraldehyde-butanol dehydrogenase gene:

DNA fragment consisting of SEQ ID NO: 1 from *Thermoanaerobacter mathranii*
DNA fragment consisting of SEQ ID NO: 3 from *Fonticella tunisiensis*
DNA fragment consisting of SEQ ID NO: 5 from *Thermoclostridium caenicola*
DNA fragment consisting of SEQ ID NO: 7 from *Thermoanaerobacter ethanolicus*
DNA fragment consisting of SEQ ID NO: 9 from *Bacillus coagulans*

### Butyraldehyde dehydrogenase gene:

DNA fragment consisting of SEQ ID NO: 11 from *Thermus thermophilus*

### Butanol dehydrogenase gene:

DNA fragment consisting of SEQ ID NO: 13 from *Bacillus coagulans*
DNA fragment consisting of SEQ ID NO: 15 from *Zymomonas mobilis* subsp. *mobilis*
DNA fragment consisting of SEQ ID NO: 17 from *Thermoanaerobacter thermocopriae*
DNA fragment consisting of SEQ ID NO: 19 from *Geobacillus stearothermophilus*

The primers shown below were used for PCR. Each primer contains a sequence homologous to that contained in vector pCAMO-6.

### Primers for amplification of butyraldehyde-butanol dehydrogenase gene of Thermoanaerobacter mathranii (SEQ ID NO: 1)

(a-11) 5'-ATGAAAGTAACAAATGCATGCCTACTTTATTACAAG-3' (SEQ ID NO: 41)
(b-11) 5'-GTCGACGGAGCTCGAATTCTTATTCTCCATAGGCTTTTC-3' (SEQ ID NO: 42)

### Primers for amplification of butyraldehyde-butanol dehydrogenase gene of Fonticella tunisiensis (SEQ ID NO: 3)

(a-12) 5'-ATCTGGAGGAGAAACGCATATGAAAGTAACAAATGCAGAAGAAC-3' (SEQ ID NO: 43)
(b-12) 5'-GTCGACGGAGCTCGAATTCTTATTTATTATTTTCTTCAGTTTCC-3' (SEQ ID NO: 44)

### Primers for amplification of butyraldehyde-butanol dehydrogenase gene of Thermoclostridium caenicola (SEQ ID NO: 5)

(a-13) 5'-ATCTGGAGGAGAAACGCATATGGAAAACAAAGAAAGAAAGATTG-3' (SEQ ID NO: 45)
(b-13) 5'-GTCGACGGAGCTCGAATTCTCATGCTCTCTTCGCTCCGACTCTG-3' (SEQ ID NO: 46)

### Primers for amplification of butyraldehyde-butanol dehydrogenase gene of Thermoanaerobacter ethanolicus (SEQ ID NO: 7)

(a-14) 5'-ATGAAAGTAACAAATGCATGCCTAACTTATTACAAGAAAGAAG-3' (SEQ ID NO: 47)
(b-14) 5'-GTCGACGGAGCTCGAATTCTTATTCTCCATAGGCTTTTC-3' (SEQ ID NO: 48)

### Primers for amplification of butyraldehyde-butanol dehydrogenase gene of Bacillus coagulans (SEQ ID NO: 9)

(a-15) 5'-ATCTGGAGGAGAAACGCATATGGCAATCGATGAAAAAGTTGTTGG-3' (SEQ ID NO: 49)
(b-15) 5'-GTCGACGGAGCTCGAATTCTTATTTTGCTTCAACGCCTTC-3' (SEQ ID NO: 50)

### Primers for amplification of butyraldehyde dehydrogenase gene of Thermus thermophilus (SEQ ID NO: 11)

(a-16) 5'-ATCTGGAGGAGAAACGCATATGTCCGAAAGGGTTAAGGTAGCC-3' (SEQ ID NO: 51)
(b-16) 5'-GTCGACGGAGCTCGAATTCTCACGCCACCACCTCCTCCACGGG-3' (SEQ ID NO: 52)

### Primers for amplification of butanol dehydrogenase gene of Bacillus coagulans (SEQ ID NO: 13)

(a-17) 5'-ATCTGGAGGAGAAACGCATATGAGAGCAGCAGTCGTCAGTTCTG-3' (SEQ ID NO: 53)
(b-17) 5'-GTCGACGGAGCTCGAATTCTTATTTCAACGACATATCCAGGACC-3' (SEQ ID NO: 54)

### Primers for amplification of butanol dehydrogenase gene of Zymomonas mobilis subsp. mobilis (SEQ ID NO: 15)

(a-18) 5'-GATCTGGAGGAGAAACGCATATGAAAGCAGCCGTCATAAC-3' (SEQ ID NO: 55)
(b-18) 5'-TGTCGACGGAGCTCGAATTCCTAGTGATGGGTAAAATCAA-3' (SEQ ID NO: 56)

### Primers for amplification of butanol dehydrogenase gene of Thermoanaerobacter thermocopriae (SEQ ID NO: 17)

(a-19) 5'-ATGAAAGTAACAAATGCATGCCTACCTTATTACAAGAAAAAAAGG-3' (SEQ ID NO: 57)
(b-19) 5'-GTCGACGGAGCTCGAATTCCTATATCAGAATTTCCATTATTTG-3' (SEQ ID NO: 58)

### Primers for amplification of butanol dehydrogenase gene of Geobacillus stearothermophilus (SEQ ID NO: 19)

(a-20) 5'-ATCTGGAGGAGAAACGCATATGGCTGTTGATGAAAAAGTATTAGACC-3' (SEQ ID NO: 59)
(b-20) 5'-GTCGACGGAGCTCGAATTCTCATTCAACCCCTTTATACGCTTGGCG-3' (SEQ ID NO: 60)

Each resulting PCR reaction mixture was subjected to agarose gel electrophoresis. The results showed that an about 1.0-kbp DNA fragment for the butyraldehyde dehydrogenase gene, an about 1.1-kbp DNA fragment for the butanol dehydrogenase genes, and an about 2.7-kbp DNA fragment for the butyraldehyde-butanol dehydrogenase genes were detected.

Gel portions containing the DNA fragments corresponding to these genes were cut out of the agarose gel, and the DNA fragments were recovered from the gel portions.

Plasmid vector pCAMO-6 was amplified by PCR for subsequent DNA ligation by Gibson Assembly. The primers shown below were used for PCR.

### Primers for amplification of plasmid vector pCAMO-6

(a-21) 5'-GAATTCGAGCTCCGTCGACA-3' (SEQ ID NO: 61)
(b-21) 5'-ATGCGTTTCTCCTCCAGATC-3' (SEQ ID NO: 62)

The resulting PCR reaction mixture was subjected to agarose gel electrophoresis. The results showed that an about 5.2-kbp DNA fragment corresponding to the vector gene was detected. A gel portion containing this fragment was cut out of the agarose gel, and this fragment was recovered from the gel portion.

The DNA fragments containing the dehydrogenase genes were individually ligated to the DNA fragment containing the synthesized vector pCAMO-6 using Gibson Assembly Master Mix manufactured by New England Biolabs.

*Escherichia coli* strain JM109 was transformed separately with each resulting reaction mixture by heat shock method, applied to LB medium containing 50 µg/mL kanamycin, and cultured at 37°C for 24 hours.

Strains grown on the LB medium were individually inoculated into 5 mL of liquid LB medium containing 50 µg/mL kanamycin per test tube using a platinum loop, and cultured with shaking at 37°C. Plasmid DNA was extracted from each culture liquid. The analysis of the sequences of the genes inserted in the plasmids was outsourced to Eurofins Genomics K.K. and performed by Sanger method. The results confirmed that the gene sequences are identical to the corresponding sequences in a database.

### (3) Transformants of Hydrogenophilus thermoluteolus

### (3-1) Gene transfer

*Hydrogenophilus thermoluteolus* strain TH-1 was transformed separately with each obtained plasmid by electric pulse method (electroporation), seeded onto solid LB medium containing 50 µg/mL kanamycin, and cultured at 52°C for 24 hours. Strains grown on the solid LB medium were individually inoculated onto solid LB medium containing 50 µg/mL kanamycin using a platinum loop, and cultured at 52°C for 24 hours. PCR was performed on the grown strains on the solid LB medium to confirm amplification of the fragments inserted in the plasmids.

The results showed that DNA fragments having lengths corresponding to the respective genes were amplified. The plasmids containing the genes from the respective bacteria, as well as the transformants of *Hydrogenophilus thermoluteolus* strain TH-1 with these plasmids, were named as shown in Table 3.

**[Table 3]**

| Bacterial strain | Introduced gene |
|---|---|
| BOH-01 | Butyraldehyde-butanol dehydrogenase gene (SEQ ID NO: 1) |
| | (*Thermoanaerobacter mathranii*) |
| BOH-02 | Butyraldehyde dehydrogenase gene (SEQ ID NO: 11) |
| | (*Thermus thermophilus*) |
| BOH-03 | Butanol dehydrogenase gene (SEQ ID NO: 13) |
| | (*Bacillus coagulans*) |
| BOH-04 | Butanol dehydrogenase gene (SEQ ID NO: 15) |
| | (*Zymomonas mobilis* subsp. *mobilis*) |
| BOH-05 | Butyraldehyde-butanol dehydrogenase gene (SEQ ID NO: 3) |
| | (*Fonticella tunisiensis*) |
| BOH-06 | Butyraldehyde-butanol dehydrogenase gene (SEQ ID NO: 5) |
| | (*Thermoclostridium caenicola*) |
| BOH-07 | Butyraldehyde-butanol dehydrogenase gene (SEQ ID NO: 7) |
| | (*Thermoanaerobacter ethanolicus*) |
| BOH-08 | Butyraldehyde-butanol dehydrogenase gene (SEQ ID NO: 9) |
| | (*Bacillus coagulans*) |
| BOH-09 | Butanol dehydrogenase gene (SEQ ID NO: 17) |
| | (*Thermoanaerobacter thermocopriae*) |
| BOH-10 | Butanol dehydrogenase gene (SEQ ID NO: 19) |
| | (*Geobacillus stearothermophilus*) |

As described above, the genes of SEQ ID NOS: 17 and 19 are known as aldehyde-alcohol dehydrogenase genes; however, each of them encodes an enzyme that do not produce butyraldehyde from butyryl-CoA and is therefore referred to as a butanol dehydrogenase gene in the present invention.

### (3-2) Measurement of butyraldehyde dehydrogenase activity and butyraldehyde-butanol dehydrogenase activity of enzymes produced by transformants

The transformed strains of *Hydrogenophilus thermoluteolus* prepared as described above were individually inoculated into liquid A medium containing 50 µg/mL kanamycin using a platinum loop, and cultured with shaking at 52°C for 24 hours while a gas mixture of H₂:O₂:CO₂ = 7.5:1:1.5 was supplied. The bacterial cells thus cultured were collected separately from 2 mL of each culture liquid by centrifugation (4°C, 5,000 g, 10 minutes). The bacterial cells were suspended in 300 µL of reaction buffer (100 mM Tris-HCl (pH 7.5), 5 µM FeSO₄, 0.2 mM DTT), disrupted by sonication, and then centrifuged (4°C, 20,000 g, 3 minutes) to obtain the supernatants of the disrupted cells.

The disrupted cell supernatants were used as crude enzyme solutions to measure butyraldehyde dehydrogenase activity. Reaction buffer was added to each crude enzyme solution, 20 mM NADH was added as a coenzyme, and 5 mM butyryl-CoA was added as a substrate. The mixtures were reacted at 52°C for 30 minutes. Each reaction mixture was analyzed with a gas chromatograph (Shimadzu Corporation: GC-2014, Polar-WAX column). The produced butyraldehyde or butanol was identified by detecting peaks having column retention times identical to those of the respective standards. This method detects butanol in the case of a bifunctional enzyme having the activity for producing butyraldehyde from butyryl-CoA and the activity for producing butanol from butyraldehyde.

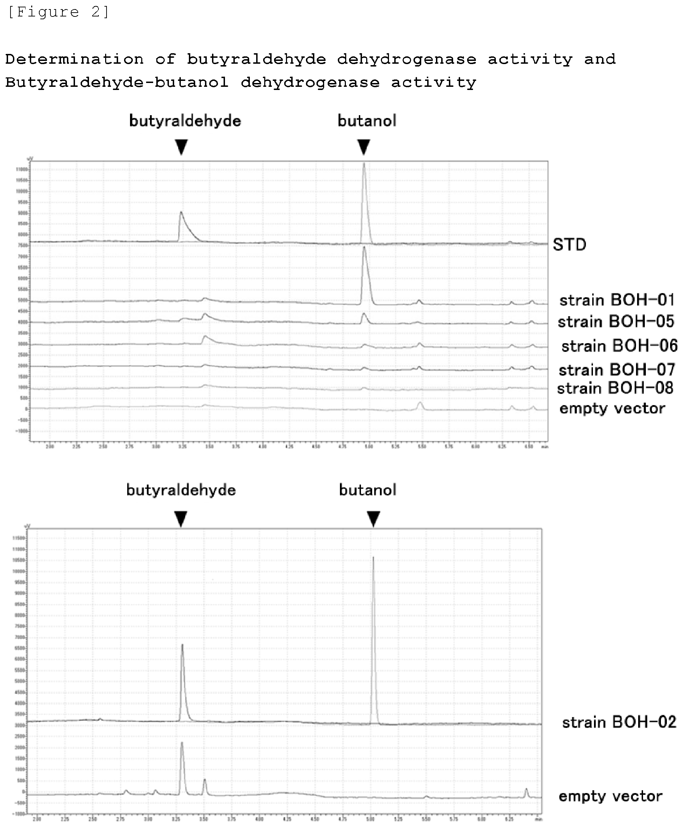

The upper panel of Fig. 2 shows a chromatogram obtained from the reaction products of butyryl-CoA with the crude enzyme solutions produced by transformed strains BOH-01, BOH-05, BOH-06, BOH-07, BOH-08, and a pCAMO-6(empty vector)-transformed control strain among the transformants shown in Table 3. Here, STD refers to the standards (2 mM each) of butanol and butyraldehyde. The enzymes produced by these transformants produced butanol using butyryl-CoA as a substrate, indicating that these transformants produced an enzyme having butyraldehyde dehydrogenase activity for producing butyraldehyde from butyryl-CoA and butanol dehydrogenase activity for producing butanol from butyraldehyde.

The lower panel of Fig. 2 shows a chromatogram obtained from the reaction product of butyryl-CoA with the crude enzyme solution produced by transformed strain BOH-02. The crude enzyme produced by strain BOH-02 produced butyraldehyde from butyryl-CoA as a substrate, but did not produce butanol, indicating that this strain produced an enzyme having butyraldehyde dehydrogenase activity.

### (3-3) Measurement of butanol dehydrogenase activity of enzymes produced by transformants

The disrupted cell supernatants of the transformed strains prepared as described above were used as crude enzyme solutions to measure butanol dehydrogenase activity. Reaction buffer was added to each crude enzyme solution, 20 mM NADH was added as a coenzyme, and 10 mM butyraldehyde was added as a substrate. The mixtures were reacted at 52°C for 30 minutes. Each reaction mixture was analyzed with a gas chromatograph (Shimadzu Corporation: GC-2014, Polar-WAX column). The produced butanol was identified by detecting a peak having a column retention time identical to that of the standard.

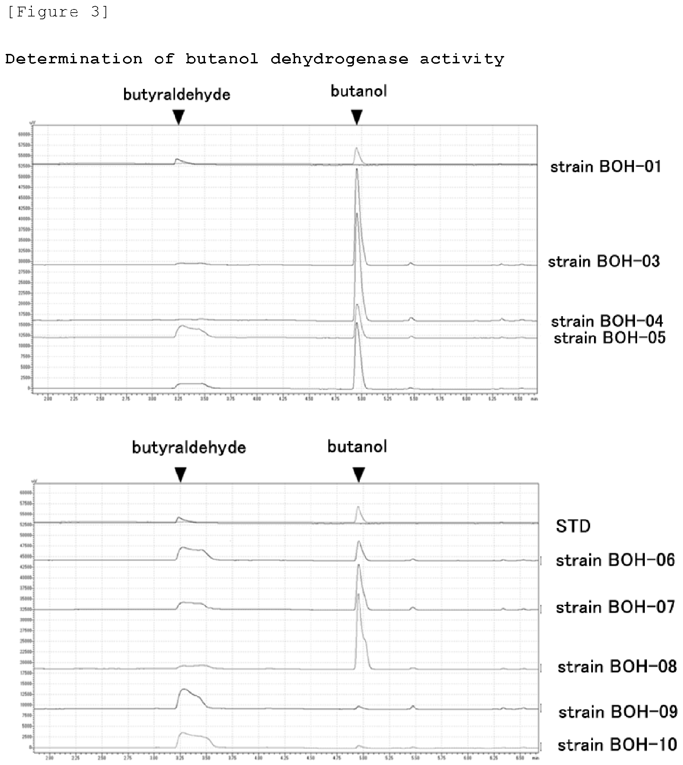

The upper and lower panels of Fig. 3 show chromatograms obtained from the reaction products of butyraldehyde with the crude enzyme solutions produced by transformed strains BOH-01, BOH-03, BOH-04, BOH-05, BOH-06, BOH-07, BOH-08, BOH-09, and BOH-10 among the transformants shown in Table 3. Here, STD refers to the standards (2 mM each) of butanol and butyraldehyde. The crude enzymes produced by these transformants produced butanol from butyraldehyde, indicating that these transformants produced an enzyme having butanol dehydrogenase activity.

### (3-4) Butanol production

Strain BOH-01 and the control strain with empty vector pCAMO-6, both prepared as described above, are individually inoculated into liquid A medium containing 50 µg/mL kanamycin using a platinum loop, and cultured with shaking at 52°C for 24 hours while a gas mixture of H₂:O₂:CO₂ = 7.5:1:1.5 is supplied. After that, culture supernatants are individually collected by centrifugation (4°C, 5,000 g, 10 minutes). In the culture supernatant of strain BOH-01, butanol is produced, but not in the culture supernatant of the control strain with the empty vector.

The *Hydrogenophilus* bacterium transformant of the present invention can be prepared by referring to the description of the Examples of this specification. Other strains mentioned in this specification are publicly available, as they are internationally deposited under the Budapest Treaty, or are possessed by organizations that furnish the strains without any restriction, or are marketed, or can be prepared by those skilled in the art based on this specification.

SEQ ID Nos: 1 to 20 are shown below.
[Chemical structure 1]
   SEQ ID NO: NO:1 (Thermoanaerobacter mathranii) 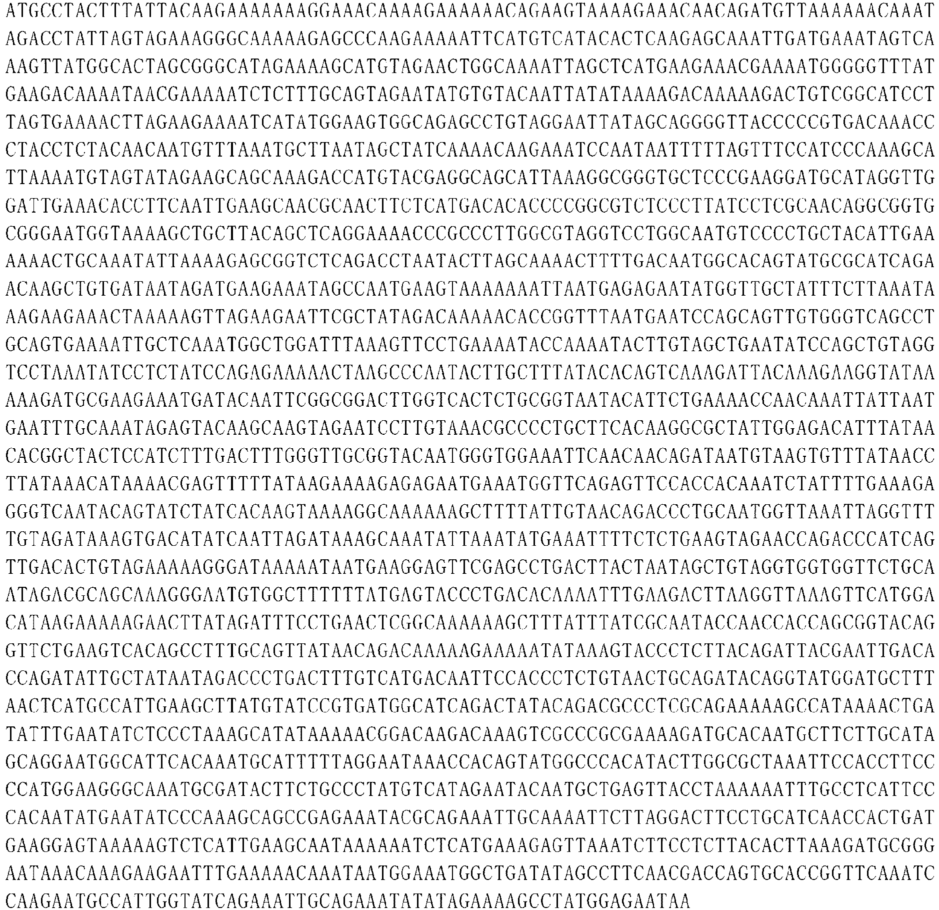
[Chemical structure 2]
   SEQ ID NO:2(Thermoanaerobacter mathranii) 
MPTLLQEKKETKEKTEVKETTDVKKQIDLLVERAKRAQEKFMSYTQEQIDEIVKVMALAG
IEKHVELAKLAHEETKMGVYEDKITKNLFAVEYVYNYIKDKKTVGILSENLEENHMEVAE
PVGIIAGVTPVTNPTSTTMFKCLIAIKTRNPIIFSFHPKALKCSIEAAKTMYEAALKAGA
PEGCIGWIETPSIEATQLLMTHPGVSLILATGGAGMVKAAYSSGKPALGVGPGNVPCYIE
KTANIKRAVSDLILSKTFDNGTVCASEQAVIIDEEIANEVKKLMREYGCYFLNKEETKKL
EEFAIDKNTGLMNPAVVGQPAVKIAQMAGFKVPENTKILVAEYPAVGPKYPLSREKLSPI
LALYTVKDYKEGIKRCEEMIQFGGLGHSAVIHSENQQIINEFANRVQASRILVNAPASQG
AIGDIYNTATPSLTLGCGTMGGNSTTDNVSVYNLINIKRVFIRKERMKWFRVPPQIYFER
GSIQYLSQVKGKKAFIVTDPAMVKLGFVDKVTYQLDKANIKYEIFSEVEPDPSVDTVEKG
IKIMKEFEPDLLIAVGGGSAIDAAKGMWLFYEYPDTKFEDLRLKFMDIRKRTYRFPELGK
KALFIAIPTTSGTGSEVTAFAVITDKKKNIKYPLTDYELTPDIAIIDPDFVMTIPPSVTA
DTGMDALTHAIEAYVSVMASDYTDALAEKAIKLIFEYLPKAYKNGQDKVAREKMHNASCI
AGMAFTNAFLGINHSMAHILGAKFHLPHGRANAILLPYVIEYNAELPKKFASFPQYEYPK
AAEKYAEIAKFLGLPASTTDEGVKSLIEAIKNLMKELNLPLTLKDAGINKEEFEKQIMEM
ADIAFNDQCTGSNPRMPLVSEIAEIYRKAYGE
[Chemical structure 3]
   SEQ ID NO:3(Fonticella tunisiensis) 
ATGAAAGTAACAAATGCAGAAGAACTAAGATTAAAGCTTGAGGAGGTTAGAAAAGCCCAAAAAATATTCGCCTCCTACAC
CCAGGAACAGGTAGATGAAATATTTAGACAGGCTGCTATGGCTGCTAACAATGCAAGAATTAAGCTTGCAAAGATGGCTG
TTGAAGAAACAGGAATGGGAATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTTGCAGAACCTATAGGAGTTAT
CGCTGCAATAGTACCAACTACCAACCCGACATCCACTGCAATATTTAAAGCATTAATCGCTTTAAAAACAAGAAACGGTG
TGATTTTCTCACCCCATCCAAGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATCGTTATGAAGGAAGCTGATAT
GATACTTGCCACAGGCGGCCCCGGAATGGTAAGAGCTGCTTATTCCTCAGGAAAACCCGCAATAGGAGTAGGTGCTGGAA
ATACGCCTGCAATAATAGATGAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTTAAGCGTGAGTTTGAGGAAAG
AGGAGCATACTTCCTAAAGGGAGAGGAAATCGACAAGGTTAGAAAGATAATACTTGTTAATGGTTCAATAAATGCAAATA
TAGTTGGACAGCCTGCCCACAAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCTATGTATAAGGCTAAAACCTT
CGATGAGGCACTTCAGAAAGCTGCAAGGCTTGTGGAACTTGGCGGATTTGGGCATACATCAGTTCTATATACCGACCAGG
TAAAATCAAGGGATAGAATTGAAAAATTTGGTGCGACTATGAAGACAGGAAGAACAATAATCAATATGCCTTCGTCCCAA
GGTGCAATAGGAGATATCTATAACTTCAGGCTTGCTCCTTCGCTAACACTCGGATGTGGTACATGGGGTGGAAACTCTGT
ATCTGAAAACGTTGGAGTTAAGCATCTTTTAAACATCAAGAGTGTTGCTGAAAGGAGAGAAAATATGCTTTGGTTCAGAG
TTCCTGAAAAGATATACTTCAAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACCCTTGATGAAATAGGAATTGA
ATATAGCGTGTTCTTTGACGTTGAACCAGACCCAACACTTGCAACTGCAAGAAAGGGTGCAGCACAAATGAAAAGCCTCA
ATCCGGATGTAATAATTGCAGTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAGTTCCCAAAGATGGGCGAAAA
GGCTATGATGGTTTCAATCCCAACTTCTTCCGGAACAGGTTCAGAGGTTACTCCATTTGCTGTTATAACCGATGAAAAGA
CGGGAATTAAATATCCTCTTGCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTATGTTTCGGTACTTGCATCAGA
GTATACTAACGGACTTGCCCTTGAAGCGATAAGACTTATATTCAAATATCTACCACAGGCATATAATGATGGTCCAAACA
ACATCAAAGCAAGGGAAAAGATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGCCTTCTAATTAACGAAGTAAT
AAGATTTAATGCAGTTGATAACCCAAGAAAGCAAACTGCATTCCCACAGTATAAATATCCAAACGCCAAGTTCAGATATG
CAAGGATTGCTGACTACCTGAACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAATTCTACGCAACCCTTGATGA
AATGTGCGAACAGGCCTTCGACGACCAGTGCACAGGTGCCAACCCAAGATATCCGTTAATAAGCGAAATCAAGCAGATGT
ATATAAACGCTTTCGAAGGAATTTCAAAGGAAACTGAAGAAAATAATAAATAA
[Chemical structure 4]
   SEQ ID NO:4(Fonticella tunisiensis) 
MKVTNAEELRLKLEEVRKAQKIFASYTQEQVDEIFRQAAMAANNARIKLAKMAVEETGMG
IVEDKVIKNHFASEYIYNKYKDEKTCGVIDRDESFGIMRVAEPIGVIAAIVPTTNPTSTA
IFKALIALKTRNGVIFSPHPRAKKSTIAAAKIVLDAAVKAGAPEGIIGWIDEPTVELSQI
VMKEADMILATGGPGMVRAAYSSGKPAIGVGAGNTPAIIDETAHIKMAVSSILLSKTFDN
GVICASEQSVIVMEQIYDEVKREFEERGAYFLKGEEIDKVRKIILVNGSINANIVGQPAH
KIAEMAGVKVPETAKVLIGEVESVELDEPFSHEKLSPVLAMYKAKTFDEALQKAARLVEL
GGFGHTSVLYTDQVKSRDRIEKFGATMKTGRTIINMPSSQGAIGDIYNFRLAPSLTLGCG
TWGGNSVSENVGVKHLLNIKSVAERRENMLWFRVPEKIYFKYGCLPVALKELKDMGKKRA
FIVTDKVLYNLGFIDTVTKTLDEIGIEYSVFFDVEPDPTLATARKGAAQMKSLNPDVIIA
VGGGSPMDAAKIMWVLYEHPEVRFEDLAMRFMDIRKRVYQFPKMGEKAMMVSIPTSSGTG
SEVTPFAVITDEKTGIKYPLADYELTPDMAIVDAELMLNMPKGLTAASGIDALTHAIEAY
VSVLASEYTNGLALEAIRLIFKYLPQAYNDGPNNIKAREKMAHAATMAGMAFANAFLGVC
HSMAHKLGAMHHVPHGVANGLLINEVIRFNAVDNPRKQTAFPQYKYPNAKFRYARIADYL
NLGGNTEDEKIELLIKAIDDLKNQVNMPKTISEAGVSREKFYATLDEMCEQAFDDQCTGA
NPRYPLISEIKQMYINAFEGISKETEENNK
[Chemical structure 5]
   SEQ ID NO:5(Thermoclostridium caenicola) 
ATGGAAAACAAAGAAAGAAAGATTGTAGACAGTGTGGACGCGTTAAAGAAAGTCATTGCCCGGGTAAGGGAAGCGCAAAGGAAATTTTCAACCTA
TTCCCAGGAACAGGTGGATGAAATTTTTAAGGCTGCTGCCACTGCCGCAAACCAGGCAAGAATTCCGCTGGCGAAAATGGCGGTGGAAGAAACCG
GTATGGGCGTTGTGGAAGATAAAGTTATAAAAAACCACTATGCCGCCGAATATGTTTACAATACGTATAAGAACGTAAGAACCTGCGGGGTTTTG
GAAGAAGATAAGGCATACGGTATCAAAAAAATTGCCGAGCCTGTCGGTGTGATTGCCGCTGTTATTCCTACCACTAACCCTACTTCCACGGCAAT
TTTTAAAACCCTGATCGCGCTGAAAACACGCAATGCCGTTATTATCAGCCCACATCCGAGAGCTAAAAAATGCACCATCGCTGCGGCAAAAATCG
TATTGGATGCTGCCGTTGCCGCGGGGGCTCCGGAAGGGATTATTGACTGGATTGATGTGCCGAACATTGAGTTGACCAATCTGCTTATGAAAGAA
GCAGACATTATCCTTGCCACAGGTGGTCCCGGTATGGTGAAGGCGGCTTATTCCTCCGGGAAACCGGCTATCGGTGTCGGGCCTGGCAATACTCC
GGCCGTTATTGATGAAAGCGCCGACGTTGTCCTTGCCGTGAACTCCATTATTCATTCCAAAACGTTTGATAATGGTATGATTTGCGCATCAGAAC
AATCGGTAATTGTTCACAAGAATGTTTATGACGCTGTAAAAGCTGAATTTGCGGCCCGTGGATGCTATTTCCTTAAAGAAGATGAAATTGAAAAG
GTACGCAAGACGATTTTTGTCAACGGCGCGTTAAATGCCCAGATTGTTGGTCAGCCGGCATACAAAATTGCCGAACTTGCCGGTGTGAAAGTGCC
TGTAGGCACAAAAATCCTCATCGGAGAAGTTGACAGCACAGACCCGTCGGAAGAATTTGCCCATGAAAAGCTGTCACCTGTCCTTGCGATGTATA
AAGCTGAGGATATTCATGACGCTTTTGAGAAAGCCGAACGTTTGGTTGCCAACGGAGGTTACGGTCATACCGCAGCAATTTATTTGGATGTAACC
ACCGAACAGGCTAAAATTGACGAATTCGCTTCGCGTTTGAAGACCTGCCGTATACTTGTGAATACTCCGTCTTCTCAGGGAGCTATCGGCGACAT
TTACAACTTTAAGCTTGCGCCGTCGTTAACTCTTGGCTGTGGTTCCTGGGGCGGAAATTCGGTTTCCGAAAATGTTGGGGTAAAACACCTGCTTA
ATATCAAAACGGTTGTTGAAAGAAGGGAAAATATGCTGTGGTTCCGCGCTCCCGAAAAAATATACATCAAAAAGGGATGTTTGCCGGTAGCCCTT
AATGAGTTGAAATACGTCATGGGTAAGAAACGCGCTTTTATAGTTACCGACAGCTTCCTCTATCACAATGGTTACACAAAGCCCATTACCGACAG
ACTTGACGAACTGGGCATTGAACATACAACGTTTTTTGATGTTTTACCCGATCCGACGCTTTCCTGCGCAAAAGCGGGAGCTGAGCAGATGAGAG
CCTTCCAGCCCGATACCATTATTGCCTTCGGTGGCGGATCTGCGATGGACGCCGCGAAAATCATGTGGGTAATGTATGAGCATCCCGAAGTTGAT
TTCATGGACATGGCTATGCGTTTTATGGATATCAGGAAAAGGGTTTATACATTTCCGAAAATGGGAGAAAAGGCGTATTTTGTTGCAATACCGAC
TTCTGCGGGTACCGGATCCGAAGTAACTCCTTTTGCGGTTATCACCGACGAAAAGAGTGGAGTAAAATATCCCCTCGCAGACTACGAGTTGCTCC
CCGACATGGCAATTATAGATGTGGATTTTCACATGAGTGCTCCCAAGGGCCTTACCGCCGCAGCAGGTATAGATGCCCTCACCCATGCGCTTGAG
GCGTACGTTTCTCTTATGGCCACTGATTTTACCGACGGACTGGCGAAACAGGCTCTGCAGATGATTTTTAAATACCTGCCCCGCGCCTATGATAA
CGGTCAGACCGATGTGGAAGCACGTGAAAAAATGGCTAATGCCGCCACCATTGCAGGTATGGCGTTTGCAAATGCTTTCCTTGGTGTTTGCCATT
CCATGGCACATAAGCTGGGCGCTTTTTATCATTTGCCTCATGGTATTGCCAACGCGCTTATGATAGAAGAAGTAATCCGTTTCAACGCTTCGGAA
GCACCGGCCAAAATGGGAACTTTCCCGCAATACGGATACCCGCACGCTTTAAGACGTTATGCCGAAATTGCTGAGGCGCTTAATCTCGGCGGCGA
AAACGATCATGAAAAGCTGGAAAATCTCATTAAAGCCATTAATGATCTGAAGGCAAGGGTGGGGATCAAGTCATCAATCAAAGAGTATGGCATAA
ATGAGAAGGAATTCCTGGAACGCCTCGACGAGATGGTTGAACAGGCATTCGACGACCAGTGCACCGGTACCAATCCGCGATATCCGCTGATGAGC
GAAATAAGGCAAATGTATCTGAATGCTTATTACGGCAAAAAACATTTTGTCGATGAAGAAAAGCCAAATGAAGCGGATATCGCCGGGACAGAGGA
TGAAAACAAAGTTAAAAAGGCGTATCTGAGAGCCAGAGTCGGAGCGAAGAGAGCATGA
[Chemical structure 6]
   SEQ ID NO:6(Thermoclostridium caenicola) 
MENKERKIVDSVDALKKVIARVREAQRKFSTYSQEQVDEIFKAAATAANQARIPLAKMAV
EETGMGVVEDKVIKNHYAAEYVYNTYKNVRTCGVLEEDKAYGIKKIAEPVGVIAAVIPTT
NPTSTAIFKTLIALKTRNAVIISPHPRAKKCTIAAAKIVLDAAVAAGAPEGIIDWIDVPN
IELTNLLMKEADIILATGGPGMVKAAYSSGKPAIGVGPGNTPAVIDESADVVLAVNSIIH
SKTFDNGMICASEQSVIVHKNVYDAVKAEFAARGCYFLKEDEIEKVRKTIFVNGALNAQI
VGQPAYKIAELAGVKVPVGTKILIGEVDSTDPSEEFAHEKLSPVLAMYKAEDIHDAFEKA
ERLVANGGYGHTAAIYLDVTTEQAKIDEFASRLKTCRILVNTPSSQGAIGDIYNFKLAPS
LTLGCGSWGGNSVSENVGVKHLLNIKTVVERRENMLWFRAPEKIYIKKGCLPVALNELKY
VMGKKRAFIVTDSFLYHNGYTKPITDRLDELGIEHTTFFDVLPDPTLSCAKAGAEQMRAF
QPDTIIAFGGGSAMDAAKIMWVMYEHPEVDFMDMAMRFMDIRKRVYTFPKMGEKAYFVAI
PTSAGTGSEVTPFAVITDEKSGVKYPLADYELLPDMAIIDVDFHMSAPKGLTAAAGIDAL
THALEAYVSLMATDFTDGLAKQALQMIFKYLPRAYDNGQTDVEAREKMANAATIAGMAFA
NAFLGVCHSMAHKLGAFYHLPHGIANALMIEEVIRFNASEAPAKMGTFPQYGYPHALRRY
AEIAEALNLGGENDHEKLENLIKAINDLKARVGIKSSIKEYGINEKEFLERLDEMVEQAF
DDQCTGTNPRYPLMSEIRQMYLNAYYGKKHFVDEEKPNEADIAGTEDENKVKKAYLRARV
GAKRA
[Chemical structure 7]
   SEQ ID NO:7(Thermoanaerobacter ethanolicus) 
ATGCCTAACTTATTACAAGAAAGAAGAGAAGTAAAAGAAAAAACAGAAGTGAAAGAAACGCTGGATGTTAAAAGACAAAT
AGACCAATTAGTAGAAAAAGCACAAAGAGCACAAGAAAAATTCATGTCTTATACCCAAGAACAAATAGATAAAATAGTTA
AAGCTATGGCTTTGGCTGGTATTGAAAATCATGTGAGATTGGCAAAATTAGCCCATGAAGAAACAAAAATGGGTGTTTAT
GAAGACAAAATAACGAAAAATCTTTTTGCAGTAGAATATGTGTACAATTATATAAAAGACAAAAAGACTGTCGGCGTCCT
TAGTGAAAACTTAGAAGAAAATTATATGGAAGTGGCAGAACCTGTAGGAGTTATAGCCGGCGTAACACCCGTTACAAACC
CTACTTCTACAACAATGTTCAAAAGCTTAATCGCTATTAAAACAAGAAATCCAATAATTTTTAGCTTTCACCCAAAAGCA
TTAAAATGTAGTATAGAAACAGCAAAGACCATGTACGAGGCAGCATTAAAAGCTGGTGCACCAGAAGGATGCATAGGTTG
GATTGAAACGCCTTCAATTGAAGCAACGCAACTTCTCATGACACATCCCGGCGTCTCCCTTATCCTCGCAACAGGCGGCG
CTGGAATGGTAAAAGCCGCTTACAGCTCAGGAAAACCCGCCCTTGGCGTAGGTCCTGGCAATGTCCCCTGCTACATTGAA
AAAACTGCAAATATTAAAAGAGCAGTCTCAGACCTAATACTTAGCAAAACTTTTGACAATGGCACAGTATGCGCATCAGA
ACAAGCTGTGATAATAGACGAAGAAATAGCAGATGAAGTGAAAAAAAATATGAAAGAATATGGTTGCTATTTTCTCAATA
AAGAAGAGACTAAAATGCTAGAAGAATTCGCTATAGACAAAAACACCGGTTTAATGAATCCAGCAGTTGTAGGTCAACCG
GCAACAAAAATTGCTGAAATGGCTGGATTTAAAGTACCTGAAAACACAAAAATACTTGTAGCTGAATATCCTGCTGTAGG
ACCAGAATATCCTCTCTCCAGAGAAAAGCTAAGTCCAATACTTGCTTTATACACAGTCAAAGATTACAACGAAGGCATAA
AAAGATGTGAAGAAATGACAGAATTCGGCGGACTTGGTCACTCTGCAGTAATACATTCTGAAAACCAACAAATTATTAAT
GAATTTGCAAAAAGAGTACGAGCAGGAAGAATTCTTGTAAACGCCCCTGCTTCTCAAGGAGCTATTGGAGATATTTATAA
CACGGCTATCCCATCTTTGACTTTGGGTTGCGGTACAATGGGTAGAAATTCAACAACAGATAATGTAAGTGTTTATAACC
TTATAAACATAAAACGAGTTTTTATAAGAAAAGAGAGAATGAAATGGTTCAGAGTTCCACCACAAATTTATTTTGAAAGA
GGGTCATTACAATATCTGTCACAAGTAAAAGGCAAAAAAGCCTTTATTGTAACAGACCCTGTGATGGTTAAATTAGGTTT
TGTAGATAAAGTAACGTATCAATTAGATAAGGCAAATATTAAATATGAAATATTCTCTGAAGTTGAACCAGATCCTTCAG
TTGACACTGTAGAAAAAGGGGTAAAAATAATGAGAGGGTTTGAGCCTGACTTACTAATAGCTGTAGGTGGTGGTTCTGCA
ATAGATGCAGCGAAGGGAATGTGGCTTTTCTACGAATACCCTGATACAAAATTCGAAGACTTAAGGTTAAAATTTATGGA
CATAAGAAAAAGAACTTATAGATTTCCTGAACTGGGGAAAAAAGCTTTATTTATCGCAATACCAACCACCAGCGGTACAG
GTTCTGAAGTTACTGCCTTTGCAGTTATAACAGACAAAAAGAAAAATATAAAGTACCCTCTCACAGATTACGAATTAACA
CCAGATATCGCTATAATAGACCCTGACCTCACAATGACAATACCACCCTCTGTCACAGCAGACACTGGTATGGACGCTTT
AACCCATGCTATTGAAGCCTATGTATCTGTAATGGCATCAGACTATACAGACGCTCTCGCAGAAAAAGCTATAAAATTGA
TATTTGAATATCTACCTAAAGCATACAAAAACGGGCAAGATAAAGTCGCCCGCGAAAAGATGCACAATGCTTCATGCATA
GCTGGAATGGCCTTCACAAATGCATTTTTAGGAATAAACCATAGTATGGCTCATATACTTGGTGCTAAATTCCATCTTCC
ACACGGAAGAGCAAATGCAATACTTCTGCCCTATGTCATAGAATACAATGCTGAGTTACCTAAAAAATTTGCTTCATTCC
CACAATATGAATATCCCAAAGCAGCCGAGAAATATGCAGAAATTGCAAAATTCTTGGGACTTCCTGCATCAACCATTGAA
GAAGGAGTAAAAAGCCTCATTGAAGCAATAAAAAATCTTATGAAAGAGCTTAATCTTCCTTTGACACTTAAAGAAGCAGG
CATAAATAAAGAAGAATTCGAAAAACAAATAATGGAAATGTCTGATATAGCCTTTAATGACCAGTGCACCGGTTCAAACC
CAAGAATGCCATTGGTATCCGAAATTGCAGAAATATATAGAAAAGCCTATGGAGAATAA
[Chemical structure 8]
   SEQ ID NO:8(Thermoanaerobacter ethanolicus) 
MPNLLQERREVKEKTEVKETLDVKRQIDQLVEKAQRAQEKFMSYTQEQIDKIVKAMALAG
IENHVRLAKLAHEETKMGVYEDKITKNLFAVEYVYNYIKDKKTVGVLSENLEENYMEVAE
PVGVIAGVTPVTNPTSTTMFKSLIAIKTRNPIIFSFHPKALKCSIETAKTMYEAALKAGA
PEGCIGWIETPSIEATQLLMTHPGVSLILATGGAGMVKAAYSSGKPALGVGPGNVPCYIE
KTANIKRAVSDLILSKTFDNGTVCASEQAVIIDEEIADEVKKNMKEYGCYFLNKEETKML
EEFAIDKNTGLMNPAVVGQPATKIAEMAGFKVPENTKILVAEYPAVGPEYPLSREKLSPI
LALYTVKDYNEGIKRCEEMTEFGGLGHSAVIHSENQQIINEFAKRVRAGRILVNAPASQG
AIGDIYNTAIPSLTLGCGTMGRNSTTDNVSVYNLINIKRVFIRKERMKWFRVPPQIYFER
GSLQYLSQVKGKKAFIVTDPVMVKLGFVDKVTYQLDKANIKYEIFSEVEPDPSVDTVEKG
VKIMRGFEPDLLIAVGGGSAIDAAKGMWLFYEYPDTKFEDLRLKFMDIRKRTYRFPELGK
KALFIAIPTTSGTGSEVTAFAVITDKKKNIKYPLTDYELTPDIAIIDPDLTMTIPPSVTA
DTGMDALTHAIEAYVSVMASDYTDALAEKAIKLIFEYLPKAYKNGQDKVAREKMHNASCI
AGMAFTNAFLGINHSMAHILGAKFHLPHGRANAILLPYVIEYNAELPKKFASFPQYEYPK
AAEKYAEIAKFLGLPASTIEEGVKSLIEAIKNLMKELNLPLTLKEAGINKEEFEKQIMEM
SDIAFNDQCTGSNPRMPLVSEIAEIYRKAYGE
[Chemical structure 9]
   SEQ ID NO:9(Bacillus coagulans) 
ATGGCAATCGATGAAAAAGTTGTTGGCAAATCTGCGCAAATCACGGAAATGGTCGATTCCCTCGTCGCAAAAGGGCAAAA
AGCACTGCGTGAATTTATGGAACTGGATCAGGCACAAGTGGACAATATCGTAAAACAAATGGCACTTGCCGGGTTGGAAC
AGCATATGGTGCTCGCCAAAATGGCCGTGGAAGAAACAGGGCGCGGCGTATATGAAGACAAAATGACAAAAAACTTGTTT
GCAACAGAATACATCTATCACAACATTAAATATAACAAAACAGTCGGCATCATTGACGAAAACAATAATGAAGGGATTGT
TAAATTTGCGGAACCGGTCGGCGTGATTGCCGGGGTCACTCCTGTAACAAACCCGACATCAACCACGATGTTTAAAGCAT
TAATTGCCATTAAAACACGCAACCCAATTATTTTCGCGTTCCATCCGTCCGCACAAAAATGCAGCAGCCATGCCGCAAAA
GTGATGCTTGACGCTGCTGTGAAAGCCGGCGCCCCGGAAAACTGCATCCAATGGATCGAAAAACCGGCCATTGAAGCGAC
ACAGCAATTGATGAACCATTCGGGTATTTCACTGATTCTTGCAACGGGCGGTTCCGGCATGGTAAAGTCCGCATATTCGA
GCGGGAAACCTGCGCTTGGCGTCGGGCCGGGAAACGTCCCTTGCTATATTGAAAAATCTGCTGATATCAAACGCGCCGTC
AATGATTTGATTTTGTCCAAAACGTTTGACAACGGCATGATCTGTGCGTCGGAACAGGCTGTCATCATCGACAAAGAAAT
TTATGACAATGTGAAAAACGAATTGATTGCAAATCAATGCTACTTCCTGAATGAAGCGGAGAAGAAAAAGGTTGAGAAAA
CCGTCATCAATGAAAAGACGCAATCCGTCAACAGCGCCATTGTCGGCAAACCGGCCTATGAAATTGCGAAAATGGCAGGC
GTAAATGTTCCGGAAGATACCAAAATTTTAATCGCCGAACTTACAGGTGTCGGCCCGGACTACCCGCTTTCCAGAGAAAA
ATTAAGCCCGGTTTTGGCCTGTTACAAAGCCAACAGCACAAAACAAGGTTTCGAATTTGCAGAAGCCATGCTCGAATTCG
GCGGACTTGGTCACTCGGCTGTGATCCATTCGACAAACGATGAAGTCATTGAACAATACGGCTTAAAAATGAAAGCCGGC
CGGATCATTGTCAACTCCCCGTCTTCGCAAGGGGCAATCGGCGACATTTACAATGCCTACATGCCTTCTTTGACACTCGG
CTGCGGCACGTTTGGCGGCAACTCGGTGTCCACAAACGTCGGTGTCATCAATCTTTACAATGTAAAAACAATGGCGAAAC
GGAGAGTCAATATGCAATGGTTTAAAATTCCACCGCGCGTGTATTTTGAAAAGAACTCGGTCCAATACCTGGAAAAAATG
CCGGATATCTCAAGTGCCTTTATTGTCACCGATCCGGATATGGTCAGGCTTGGATTCGTTGAGAAAGTACTGTATTACTT
GCGGAAACGCCCGGACTATGTGCACTGTGAAATTTTCTCAGAAGTCGAACCGGATCCGTCTATTGAAACGGTTCGGAAAG
GTGCCGCAGCTATGGCAAGCTTCCAGCCGGATGTCATTATCGCGCTTGGCGGCGGGTCGGCGATGGATGCTGCAAAAGGC
ATGTGGCTGTTCTATGAGCATCCGGAAGTTGATTTCAACGAATTGAAGCAAAAATTTATGGACATCCGGAAACGGGTTGC
CAAATTCCCGAAACTGGGTGAAAAAGCGCAGCTGGTCTGCATTCCGACCACATCGGGCACCGGTTCTGAAGTGACTTCTT
TCGCCGTCATTTCGGATAAAAAGAACAACACAAAATATCCGCTCGCAGACTATGAACTGACACCGGATGTTGCGATCATT
GACCCGCAGTTTGTTATGACCGTTCCGAAATCCGTAACAGCCGATACCGGGATGGACGTTTTGACCCATGCGATTGAAGC
CTATGTATCCAACATGGCCAACGACTATACGGACGGCCTGGCAATTAAGGCCATCCAGCTTGTTCACGAATACTTGCCGA
AAGCATACGCAAATGGCAATGATGCACTGGCACGCGAAAAAATGCACAATGCATCGACTTTGGCAGGGATGGCGTTCTCG
AATGCATTCCTTGGCATCAACCATTCACTTGCCCATAAAATCGGCGCTGAATTCCATATTCCGCACGGCCGGGCTAACGC
GATTCTGTTGCCGCATGTCATCCGCTATAACGCGCAAAAGCCGAAAAAATTTGCGGCATTCCCGAAATACGAACATTTTA
TTGCGGATCAACGCTACGCGGAAATCGCGCGCGTACTTGGTCTTCCGGCAAGCACGACAGAAGAAGGTGTCGAAAGCCTT
TGCCAGGAAATCATCCGGATGTGCAAACTGTTCAATATTCCGCTTAGCCTAAAAGCAGCCGGCGTGAACCGGGCGGACTT
TGAAAAACGGGTTGCCATTATTGCAGACCGCGCATTTGAAGACCAATGCACACCTGCAAACCCTAAATTGCCGCTTGTCA
GCGAATTGGAAGACATTTTACGCAAAGCTTTTGAAGGCGTTGAAGCAAAATAA
[Chemical structure 10]
   SEQ ID NO:10(Bacillus coagulans) 
MAIDEKVVGKSAQITEMVDSLVAKGQKALREFMELDQAQVDNIVKQMALAGLEQHMVLAK
MAVEETGRGVYEDKMTKNLFATEYIYHNIKYNKTVGIIDENNNEGIVKFAEPVGVIAGVT
PVTNPTSTTMFKALIAIKTRNPIIFAFHPSAQKCSSHAAKVMLDAAVKAGAPENCIQWIE
KPAIEATQQLMNHSGISLILATGGSGMVKSAYSSGKPALGVGPGNVPCYIEKSADIKRAV
NDLILSKTFDNGMICASEQAVIIDKEIYDNVKNELIANQCYFLNEAEKKKVEKTVINEKT
QSVNSAIVGKPAYEIAKMAGVNVPEDTKILIAELTGVGPDYPLSREKLSPVLACYKANST
KQGFEFAEAMLEFGGLGHSAVIHSTNDEVIEQYGLKMKAGRIIVNSPSSQGAIGDIYNAY
MPSLTLGCGTFGGNSVSTNVGVINLYNVKTMAKRRVNMQWFKIPPRVYFEKNSVQYLEKM
PDISSAFIVTDPDMVRLGFVEKVLYYLRKRPDYVHCEIFSEVEPDPSIETVRKGAAAMAS
FQPDVIIALGGGSAMDAAKGMWLFYEHPEVDFNELKQKFMDIRKRVAKFPKLGEKAQLVC
IPTTSGTGSEVTSFAVISDKKNNTKYPLADYELTPDVAIIDPQFVMTVPKSVTADTGMDV
LTHAIEAYVSNMANDYTDGLAIKAIQLVHEYLPKAYANGNDALAREKMHNASTLAGMAFS
NAFLGINHSLAHKIGAEFHIPHGRANAILLPHVIRYNAQKPKKFAAFPKYEHFIADQRYA
EIARVLGLPASTTEEGVESLCQEIIRMCKLFNIPLSLKAAGVNRADFEKRVAIIADRAFE
DQCTPANPKLPLVSELEDILRKAFEGVEAK
[Chemical structure 11]
   SEQ ID NO:11(Thermus thermophilus) 
ATGTCCGAAAGGGTTAAGGTAGCCATCCTGGGCTCCGGCAACATCGGGACGGACCTGATGTACAAGCTCCTGAAGAACCC
GGGCCACATGGAGCTTGTGGCGGTGGTGGGGATAGACCCCAAGTCCGAGGGCCTGGCCCGGGCGCGGGCCTTAGGGTTAG
AGGCGAGCCACGAAGGGATCGCCTACATCCTGGAGAGGCCGGAGATCAAGATCGTCTTTGACGCCACCAGCGCCAAGGCC
CACGTGCGCCACGCCAAGCTCCTGAGGGAGGCGGGGAAGATCGCCATAGACCTCACGCCGGCGGCCCGGGGCCCTTACGT
GGTGCCCCCGGTGAACCTGAAGGAACACCTGGACAAGGACAACGTGAACCTCATCACCTGCGGGGGGCAGGCCACCATCC
CCCTGGTCTACGCGGTGCACCGGGTGGCCCCCGTGCTCTACGCGGAGATGGTCTCCACGGTGGCCTCCCGCTCCGCGGGC
CCCGGCACCCGGCAGAACATCGACGAGTTCACCTTCACCACCGCCCGGGGCCTGGAGGCCATCGGGGGGGCCAAGAAGGG
GAAGGCCATCATCATCCTGAACCCGGCGGAACCCCCCATCCTCATGACCAACACCGTGCGCTGCATCCCCGAGGACGAGG
GCTTTGACCGGGAGGCCGTGGTGGCGAGCGTCCGGGCCATGGAGCGGGAGGTCCAGGCCTACGTGCCCGGCTACCGCCTG
AAGGCGGACCCGGTGTTTGAGAGGCTTCCCACCCCCTGGGGGGAGCGCACCGTGGTCTCCATGCTCCTGGAGGTGGAGGG
GGCGGGGGACTATTTGCCCAAATACGCCGGCAACCTGGACATCATGACGGCTTCTGCCCGGAGGGTGGGGGAGGTCTTCG
CCCAGCACCTCCTGGGGAAGCCCGTGGAGGAGGTGGTGGCGTGA
[Chemical structure 12]
   SEQ ID NO:12(Thermus thermophilus) 
MSERVKVAILGSGNIGTDLMYKLLKNPGHMELVAVVGIDPKSEGLARARALGLEASHEGI
AYILERPEIKIVFDATSAKAHVRHAKLLREAGKIAIDLTPAARGPYVVPPVNLKEHLDKD
NVNLITCGGQATIPLVYAVHRVAPVLYAEMVSTVASRSAGPGTRQNIDEFTFTTARGLEA
IGGAKKGKAIIILNPAEPPILMTNTVRCIPEDEGFDREAVVASVRAMEREVQAYVPGYRL
KADPVFERLPTPWGERTVVSMLLEVEGAGDYLPKYAGNLDIMTASARRVGEVFAQHLLGK
PVEEVVA
[Chemical structure 13]
   SEQ ID NO:13(Bacillus coagulans) 
ATGAGAGCAGCAGTCGTCAGTTCTGATAAGCAAAAACTGGTAGAGGTCAAAGATGTGCAGTTGCGTCCACTTGAATATGG
GGAAGCTTTGGTGGATATCGAATACTGCGGTGTCTGCCATACGGATCTGCATGTGGCAAAGGGCGACTTTGGCGATGTGC
CGGGACGCATTCTCGGCCATGAAGGAGTCGGCATTGTCAGGGAAGTCGCGGATGGTGTCACTTCTCTTAAAGTGGGAGAT
CGTGTAAGCGTTGCCTGGTTTTTTGAAGGCTGCGGGCATTGTGAGTACTGTTTAACGGGAAATGAAACATTTTGCCGTGA
TGTGAAAAATGCAGGCTACAGTGTCGACGGAGCAATGGCAGAGCAGTGCATCGTCAAAGCGGATTACGCCGTAAAAGTGC
CGGAAGGGCTGGATCCAAGGCAGGCGACGAGCATTACTTGCGCAGGTGTCACCACTTATAAAGCCATTAAAGTCGCAGAT
GTGAAGCCGGGCGAATGGATTGCCATTTATGGAGCCGGCGGTCTTGGTAACCTTGCTGTCCAGTACGCCAAACATGTATT
TAATGCGAAAGTCATTGCGATCGATATCAATGACGACAAACTTTCACTGGCAAAAGAAGTCGGTGCTGATCTCACCTTTA
ATTCCAAAATGAAAGGCGATCCGGCTGAATGGATCCAAAAAGAAGTTGGCGGCGCGCATGCTGCAGTGGTAACGGCTGTT
TCAAAACAGGCTTTTAACCAGGCTGTCCACTCTGTACGGCCGGTTTCAAAAGTCGTTGCTGTCGGGCTCCCTCCGGAAAC
AATGGATCTTGAAATTGTGAAGACGGTTCTTGACGGAATAGAAGTCGTCGGCTCTTTGGTAGGCACGAGAAAAGACCTTG
AGGAAGCCTTTATGTTCGGGGCGGAAGGAAAAGTCGTCCCGGTTGTGCACACGCGCAGACTGGAAGAAGTGGGAGATGTG
TTTAAAGAAATGGAAGAAGGCAGAATACAGGGCAGGATGGTCCTGGATATGTCGTTGAAATAA
[Chemical structure 14]
   SEQ ID NO:14(Bacillus coagulans) 
MRAAVVSSDKQKLVEVKDVQLRPLEYGEALVDIEYCGVCHTDLHVAKGDFGDVPGRILGH
EGVGIVREVADGVTSLKVGDRVSVAWFFEGCGHCEYCLTGNETFCRDVKNAGYSVDGAMA
EQCIVKADYAVKVPEGLDPRQATSITCAGVTTYKAIKVADVKPGEWIAIYGAGGLGNLAV
QYAKHVFNAKVIAIDINDDKLSLAKEVGADLTFNSKMKGDPAEWIQKEVGGAHAAVVTAV
SKQAFNQAVHSVRPVSKVVAVGLPPETMDLEIVKTVLDGIEVVGSLVGTRKDLEEAFMFG
AEGKVVPVVHTRRLEEVGDVFKEMEEGRIQGRMVLDMSLK
[Chemical structure 15]
   SEQ ID NO:15(Zymomonas mobilis subsp. mobilis) 
ATGAAAGCAGCCGTCATAACTAAAGATCATACGATCGAAGTGAAAGACACCAAATTACGCCCTCTGAAATACGGGGAAGC
GCTTTTGGAAATGGAATATTGCGGGGTATGTCATACCGATCTCCACGTGAAAAACGGGGATTTTGGCGATGAAACCGGCA
GAATTACCGGCCATGAAGGCATCGGTATCGTCAAGCAGGTCGGGGAAGGGGTTACTTCTCTGAAAGTCGGTGACCGTGCC
AGTGTTGCATGGTTCTTCAAAGGCTGCGGCCATTGCGAATATTGTGTCAGTGGAAATGAAACGCTTTGCCGCAACGTTGA
AAATGCCGGTTATACGGTTGACGGCGCTATGGCAGAAGAATGCATCGTCGTTGCCGATTACTCGGTCAAAGTGCCAGATG
GTCTTGATCCTGCGGTTGCCAGCAGCATCACTTGCGCGGGTGTAACCACCTATAAAGCAGTCAAAGTTTCTCAGATACAG
CCGGGACAATGGCTGGCTATCTATGGCTTGGGCGGTTTAGGCAATCTAGCCCTTCAATATGCCAAGAATGTTTTCAACGC
CAAAGTGATCGCGATCGATGTCAATGATGAACAGCTCGCTTTTGCCAAAGAGCTGGGCGCAGATATGGTCATCAATCCGA
AAAACGAAGATGCTGCCAAAATCATTCAGGAAAAAGTCGGCGGCGCACATGCGACGGTGGTGACAGCTGTTGCCAAATCC
GCCTTTAACTCGGCTGTTGAGGCTATCCGCGCGGGTGGCCGTGTTGTCGCCGTTGGTCTGCCTCCTGAAAAAATGGATTT
GAGCATTCCTCGCTTGGTGCTTGACGGTATCGAAGTCTTAGGTTCTTTGGTCGGAACGCGGGAAGATTTGAAAGAAGCCT
TCCAGTTTGCAGCCGAAGGTAAGGTCAAACCGAAAGTCACCAAGCGTAAAGTCGAAGAAATCAACCAAATCTTTGACGAA
ATGGAACATGGTAAATTCACAGGCCGTATGGTTGTTGATTTTACCCATCACTAG
[Chemical structure 16]
   SEQ ID NO:16(Zymomonas mobilis subsp. mobilis) 
MKAAVITKDHTIEVKDTKLRPLKYGEALLEMEYCGVCHTDLHVKNGDFGDETGRITGHEG
IGIVKQVGEGVTSLKVGDRASVAWFFKGCGHCEYCVSGNETLCRNVENAGYTVDGAMAEE
CIVVADYSVKVPDGLDPAVASSITCAGVTTYKAVKVSQIQPGQWLAIYGLGGLGNLALQY
AKNVFNAKVIAIDVNDEQLAFAKELGADMVINPKNEDAAKIIQEKVGGAHATVVTAVAKS
AFNSAVEAIRAGGRVVAVGLPPEKMDLSIPRLVLDGIEVLGSLVGTREDLKEAFQFAAEG
KVKPKVTKRKVEEINQIFDEMEHGKFTGRMVVDFTHH
[Chemical structure 17]
   SEQ ID NO:17 (Thermoanaerobacter thermocopriae) 
ATGCCTACTTTATTACAAGAAAAAAAGGAAGCAAAAGAAAAAACAGAAGTAAAAGAAACAACAGATGTTAAAAAACAAAT
AGACCTATTAGTAGAAAGGGCGAAAAGAGCCCAAGAAAAATTCATGTCATACACTCAGCAGCAAATTGATGAAATAGTCA
AAGCTATGGCACTAACGGGCATAGAAAAGCATGTAGAACTGGCAAGATTAGCTCATGAAGAAACAAAAATGGGTGTTTAT
GAAGACAAAATAACGAAAAATCTCTTTGCAGTAGAATATGTGTACAATTATATAAAAGACAAAAAAACTGTAGGCATCCT
CAAAGAAAACTTAGAAGAAAATTATATGGAAGTGGCAGAGCCTGTAGGAATTATAGCCGGAGTAACACCTGTCACAAACC
CTACTTCTACAACAATGTTCAAATGCCTAATTGCTATTAAAACAAGAAATCCAATAATTTTTAGTTTCCATCCCAAAGCA
TTAAAATGTAGTATAGAAGCAGCAAAGACCATGTACGAGGCAGCATTAAAGGCGGGTGCTCCCGAAGGATGCATAGGTTG
GATTGAAACACCTTCAATTGAAGCAACGCAACTTCTCATGACACATCCCGGTGTCTCCCTTATCCTCGCAACAGGCGGTG
CAGGAATGGTAAAAGCTGCTTATAGCTCAGGAAAACCCGCCCTTGGCGTAGGTCCTGGCAATGTCCCCTGCTACATTGAA
AAAACTGCAAATATTAAAAGAGCGGTCTCAGACCTAATACTTAGCAAAACTTTTGACAATGGCACAGTATGCGCATCAGA
ACAAGCTGTGATAATAGATGAAGAAATAGCCAATGAAGTAAAAAAATTAATGAGAGAATATGGTTGCTATTTCTTAAATA
AAGAAGAAACTAAAAAGTTAGAAGAATTCGCTATAGACAAAAACACCGGTTTAATGAATCCAGCAGTTGTGGGTCAGCCT
GCAGTGAAAATTGCTCAAATGGCTGGATTTAAAGTTCCTGAAAATACCAAAATACTTGTAGCTGAATATCCAGCTGTAGG
TCCTGAATATCCTCTATCCAGAGAAAAACTAAGCCCAATACTTGCTTTATACACAGTCAAAGATTACAAAGAAGGTATAA
AAAGATGCGAAGAAATGATACAATTCGGCGGACTTGGTCACTCTGCGGTAATACATTCTGAAAACCAACAAATTATTAAT
GAATTTGCAAATAGAGTACAAGCAAGTAGAATCCTTGTAAACGCCCCTGCTTCACAAGGCGCTATTGGAAACATTTATAA
CACGGCTACTCCATCTTTGACTTTGGGTTGCGGTACAATGGGTGGAAATTCAACAACAGATAATGTAAGTGTTTATAACC
TTATAAATATAAAACGAGTTTTTATAAGAAAAGAGAGAATGAAATGGTTCAGAGTTCCACCACAAATCTATTTTGAAAGA
GGGTCAATACAGTATCTATCACAAGTAAAAGGCAAAAAAGCCTTTATTGTAACAGACCCTGCGATGGTTAAATTAGGTTT
TGTAGATAAAGTGACATATCAATTAGATAAAGCAAATATTAAATATGAAATTTTCTCTGAAGTAGAACCAGACCCATCAG
TTGACACTGTAGAAAAAGGGATAAAAATAATGAAGGAGTTCGAGCCTGACTTACTAATAGCTATAGGTGGTGGTTCTGCA
ATAGACGCAGCAAAGGGAATGTGGCTTTTTTATGAGTACCCTGACACAAAATTTGAAGACTTAAGGTTAAAGTTCATGGA
CATAAGAAAAAGAACTTATAGATTTCCTGAACTCGGCAAAAACGCTTTATTTATCGCAATACCAACCACCAGCGGTACAG
GTTCTGAAGTCACAGCCTTTGCAGTTATAACAGACAAAAAGAAAAATATAAAGTACCCTCTTACAGATTACGAATTGACA
CCAGATATTGCTATAATAGACCCTGACTTTGTCATGACAATTCCACCCTCTGTAACTGCAGATACAGGTATGGATGCTTT
AACTCATGCCATTGAAGCTTATGTATCCGTTATGGCATCAGACTATACAGACGCCCTCGCAGAAAAAGCCATAAAACTGA
TATTTGAATATCTCCCTAAAGCATATAAAAACGGACAAGACAAAGTCGCCCGCGAAAAGATGCACAATGCTTCTTGCATA
GCAGGAATGGCATTCACAAATGCATTTTTAGGAATAAACCACAGTATGGCCCACATACTTGGCGCTAAATTCCACCTTCC
CCACGGAAGGGCAAATGCGATACTTCTGCCCTATGTCATAGAATACAATGCTGAGTTACCTAAAAAATTTGCCTCATTCC
CACAATATGAATATCCCAAAGCAGCCGAGAAATACGCAGAAATTGCAAAATTCTTAGGACTTCCTGCATCAACCACTGAT
GAAGGAGTAAAAAGTCTCATTGAAGCAATAAAAAATCTCATGAAAGAGTTAAATCTTCCTCTTACACTTAAAGATGCGGG
AATAAACAAAGAAGAATTTGAAAAACAAATAATGGAAATGGCTGATATAGCCTTCAACGACCAGTGCACCGGTTCAAATC
CAAGAATGCCATTGGTATCAGAAATTGCAGAAATATATAGAAAAGCCTATGGAGAATAA
[Chemical structure 18]
   SEQ ID NO:18(Thermoanaerobacter thermocopriae) 
MPTLLQEKKEAKEKTEVKETTDVKKQIDLLVERAKRAQEKFMSYTQQQIDEIVKAMALTG
IEKHVELARLAHEETKMGVYEDKITKNLFAVEYVYNYIKDKKTVGILKENLEENYMEVAE
PVGIIAGVTPVTNPTSTTMFKCLIAIKTRNPIIFSFHPKALKCSIEAAKTMYEAALKAGA
PEGCIGWIETPSIEATQLLMTHPGVSLILATGGAGMVKAAYSSGKPALGVGPGNVPCYIE
KTANIKRAVSDLILSKTFDNGTVCASEQAVIIDEEIANEVKKLMREYGCYFLNKEETKKL
EEFAIDKNTGLMNPAVVGQPAVKIAQMAGFKVPENTKILVAEYPAVGPEYPLSREKLSPI
LALYTVKDYKEGIKRCEEMIQFGGLGHSAVIHSENQQIINEFANRVQASRILVNAPASQG
AIGNIYNTATPSLTLGCGTMGGNSTTDNVSVYNLINIKRVFIRKERMKWFRVPPQIYFER
GSIQYLSQVKGKKAFIVTDPAMVKLGFVDKVTYQLDKANIKYEIFSEVEPDPSVDTVEKG
IKIMKEFEPDLLIAIGGGSAIDAAKGMWLFYEYPDTKFEDLRLKFMDIRKRTYRFPELGK
NALFIAIPTTSGTGSEVTAFAVITDKKKNIKYPLTDYELTPDIAIIDPDFVMTIPPSVTA
DTGMDALTHAIEAYVSVMASDYTDALAEKAIKLIFEYLPKAYKNGQDKVAREKMHNASCI
AGMAFTNAFLGINHSMAHILGAKFHLPHGRANAILLPYVIEYNAELPKKFASFPQYEYPK
AAEKYAEIAKFLGLPASTTDEGVKSLIEAIKNLMKELNLPLTLKDAGINKEEFEKQIMEM
ADIAFNDQCTGSNPRMPLVSEIAEIYRKAYGE
[Chemical structure 19]
   SEQ ID NO:19(Geobacillus stearothermophilus) 
ATGGCTGTTGATGAAAAAGTATTAGACCAAAAGAAAGAAGTTACAAAAATGATCGATACGCTCGTCGCGAATGCGCAAAA
AGCGTTAAAAGCGTTTCGTGATTACGATCAAGAAGCGATTGATCATATCGTCAAGCAAATGGCGTTAGCTGGACTTGACA
AACATATGTATTTAGCGAAGTTAGCGATTGAAGAAACAAAACGTGGCGTGTATGAAGATAAAATCATTAAAAACATTTTT
GCGACAGAATACATTTATCATAACATCAAATATGATAAAACCGTCGGCGTTATTCGCGAAAATGAACAAGAAGGTATTAT
TGAAATTGCTGAGCCTGTTGGAGTCATCGCTGGGGTTACACCAGTAACAAACCCAACATCAACTACGATGTTTAAAGCGC
TTATTTCAATCAAGACGCGCAATCCAATCATTTTTGCGTTCCATCCGTCCGCGCAAAAATGCAGCAGTGAAGCGGCACGC
ATTTTACGTGATGCAGCGATTGCGGCCGGTGCGCCAGAACATTGTATTCAATGGATCGAAACACCTTCCGTTGAAGCAAC
GCAACAACTTATGCATCACCCTGGTGTTTCGCTTATTCTTGCGACAGGTGGAGCAGGCATGGTCAAAGCAGCATATAGCT
CAGGAAAACCAGCATTAGGGGTTGGACCTGGTAACGTACCTTGTTATATTGAAAAAACAGCAAATATTAAACGCGCGGTG
AACGACCTTATTCTTTCAAAAACGTTTGATAACGGAATGATTTGTGCATCTGAACAAGCGGTCATTATTGATAAAGAAAT
ATATGATGCGGTGAAAAACGAAATGATCGCAAACAAATGTTACTTTTTAAATGAAGAAGAAAAAGAAAAAGTCGAAAAGC
TTGTCATTAATGAAAATACGTGTGCAGTAAACCCGAATATCGTCGGTAAACCAGCATATGAAATTGCAAAAATGGCCGGT
GTGGACGTTCCAGTTGATACGAAAATTCTTGTCGCAGAATTAAAAAGCGTCGGTCCGCAAGAGCCGCTTTCTCGTGAAAA
ATTAAGTCCTGTATTGGCGTGCTATAAGGTGAATAGCACAGAAGAAGGGTTAAAACGAGCAGAAGAAATGCTTGAATTTG
GTGGACTTGGCCACTCCGCTGTCATTCATTCGGAAAATCAAGACGTCATTCTTGAATTCGGTAAGCGAATGAAAGCAGGA
CGAATCATCTCTAATGCACCATCTTCACAAGGAGCGATCGGGGACATTTACAACGCATACATTCCGTCATTGACGCTCGG
TTGCGGTACGTTTGGAGGCAACTCCGTTTCGACAAACGTGGGTGCGACACACTTAATTAATATTAAAAAATTAGCGAGAA
GGAATGTAAACATGCAATGGTTTAAAGTTCCACCAAAAATTTATTTTGAAAAACATGCGACACAATATTTAGCGAAAATG
CCAAACATCTCACGTGCCTTTATCGTTACAGACCCAGGTATGGTGAAGCTCGGCTATGTCGATAAAGTGTTATACTATTT
GCGTAAACGTCCAGATTATGTGCATTGCGAAATTTTCTCAGAAGTTGAACCAGATCCATCAATTGAAACAGTGATGAAAG
GTGCCGACATGATGCATGCGTTCCAGCCAGACGTCATCATCGCGCTCGGCGGTGGTTCGGCGATGGACGCGGCGAAAGCG
ATGTGGCTGTTCTATGAACATCCAAATACCGACTTTAATGGCTTAAAACAAAAGTTTTTAGATATTCGTAAACGCGTATT
TAAATATCCAAAACTCGGTCAAAAAGCGCAATTTGTCGCGATTCCAACAACATCTGGTACAGGTTCAGAAGTGACATCGT
TTGCGGTCATTACAGATAAGAAAAATAACGTCAAATATCCGCTTGCTGACTACGAGTTAACACCAGATGTCGCCATCGTT
GACCCACAATTCGTCATGACGATGCCAAAACATATTACAGCGGATACAGGTATGGACGTATTAACACATGCGATTGAAGC
GTACGTATCGAACATGGCAAACGACTACACAGACGGATTAGCACTAAAAGCCATTCAACTCGTCTTTGAATATTTGCCAC
GCGCATACAAAAACGGCAACGATGAAGTGGCGCGCGAAAAAATGCATAACGCATCAACGATTGCCGGTATGGCATTCGCG
AACGCATTTTTAGGCATTAACCATAGTTTAGCGCATAAACTTGGCGCAGAATTCCATATTCCGCACGGACGTGCGAACAC
GATTTTAATGCCGCACGTCATTCGTTATAACGCGACAAAACCGAAGAAATTTGTAGCGTTCCCTAAATATGAACATTTTA
TCGCGGATAAACGCTATGCGGAAATCGCACGCATTTTAGGATTGCCAGCACGCACAACGGAAGAAGGAGTCGAAAGCCTC
ATTCAAGCGATTATCAAGCTTGCAAAAGAGCTTGAAATGCCATTAAGCATCGCGGAAACAGGCGTCAGCAAAGAAGAGTT
TGAAAGCAAAGTCGAACAACTTGCTGAACTAGCTTTTGAAGATCAATGTACAACAGCCAACCCAAAATTACCATTAGTCA
GCGACCTTGCCGACATTTATCGCCAAGCGTATAAAGGGGTTGAATGA
[Chemical structure 20]
   SEQ ID NO:20(Geobacillus stearothermophilus)

MAVDEKVLDQKKEVTKMIDTLVANAQKALKAFRDYDQEAIDHIVKQMALAGLDKHMYLAK
LAIEETKRGVYEDKIIKNIFATEYIYHNIKYDKTVGVIRENEQEGIIEIAEPVGVIAGVT
PVTNPTSTTMFKALISIKTRNPIIFAFHPSAQKCSSEAARILRDAAIAAGAPEHCIQWIE
TPSVEATQQLMHHPGVSLILATGGAGMVKAAYSSGKPALGVGPGNVPCYIEKTANIKRAV
NDLILSKTFDNGMICASEQAVIIDKEIYDAVKNEMIANKCYFLNEEEKKKVEKLVINENT
CAVNPNIVGKPAYEIAKMAGVDVPVDTKILVAELKSVGPQEPLSREKLSPVLACYKVNST
EEGLKRAEEMLEFGGLGHSAVIHSENQDVILEFGKRMKAGRIISNAPSSQGAIGDIYNAY
IPSLTLGCGTFGGNSVSTNVGATHLINIKKLARRNVNMQWFKVPPKIYFEKHATQYLAKM
PNISRAFIVTDPGMVKLGYVDKVLYYLRKRPDYVHCEIFSEVEPDPSIETVMKGADMMHA
FQPDVIIALGGGSAMDAAKAMWLFYEHPNTDFNGLKQKFLDIRKRVFKYPKLGQKAQFVA
IPTTSGTGSEVTSFAVITDKKNNVKYPLADYELTPDVAIVDPQFVMTMPKHITADTGMDV
LTHAIEAYVSNMANDYTDGLALKAIQLVFEYLPRAYKNGNDEVAREKMHNASTIAGMAFA
NAFLGINHSLAHKLGAEFHIPHGRANTILMPHVIRYNATKPKKFVAFPKYEHFIADKRYA
EIARILGLPARTTEEGVESLIQAIIKLAKELEMPLSIAETGVSKEEFESKVEQLAELAFE
DQCTTANPKLPLVSDLADIYRQAYKGVE

### Industrial Applicability

The transformant of the present invention can highly efficiently produce butanol, which is in high demand for producing plastics, rubbers, fibers, polymers, biofuels, and other chemical products, using carbon dioxide as a sole carbon source. Therefore, this transformant can provide a solution to global warming caused by increased carbon dioxide emissions and contribute to highly efficient industrial production of chemical products.

## Claims

1. A transformant obtained by introducing, into a *Hydrogenophilus* bacterium, any one of the following genes (1) to (5).
(1) A gene encoding an enzyme having butyraldehyde dehydrogenase activity and a gene encoding an enzyme having butanol dehydrogenase activity
(2) A gene encoding a multifunctional enzyme having butyraldehyde dehydrogenase activity and butanol dehydrogenase activity
(3) A gene encoding an enzyme having butyraldehyde dehydrogenase activity and a gene encoding a multifunctional enzyme having butyraldehyde dehydrogenase activity and butanol dehydrogenase activity
(4) A gene encoding an enzyme having butanol dehydrogenase activity and a gene encoding a multifunctional enzyme having butyraldehyde dehydrogenase activity and butanol dehydrogenase activity
(5) A gene encoding an enzyme having butyraldehyde dehydrogenase activity, a gene encoding an enzyme having butanol dehydrogenase activity, and a gene encoding a multifunctional enzyme having butyraldehyde dehydrogenase activity and butanol dehydrogenase activity

2. The transformant according to claim 1, wherein the following DNA (a), (b), (c), (d), or (e) is used as the gene encoding a multifunctional enzyme having butyraldehyde dehydrogenase activity and butanol dehydrogenase activity.
(a) A DNA comprising the nucleotide sequence of SEQ ID NO: 1, 3, 5, 7, or 9
(b) A DNA comprising a nucleotide sequence that has 90% or more identity to SEQ ID NO: 1, 3, 5, 7, or 9 and encoding a polypeptide having butyraldehyde dehydrogenase activity and butanol dehydrogenase activity
(c) A DNA encoding a polypeptide comprising the amino acid sequence of SEQ ID NO: 2, 4, 6, 8, or 10
(d) A DNA encoding a polypeptide comprising an amino acid sequence that has 90% or more identity to SEQ ID NO: 2, 4, 6, 8, or 10, the polypeptide having butyraldehyde dehydrogenase activity and butanol dehydrogenase activity
(e) A DNA encoding a polypeptide comprising an amino acid sequence that has 1 to 100 amino acids deleted, substituted, inserted, or added in the amino acid sequence of SEQ ID NO: 2, 4, 6, 8, or 10, the polypeptide having butyraldehyde dehydrogenase activity and butanol dehydrogenase activity

3. The transformant according to claim 1 or 2, wherein the following DNA (f), (g), (h), (i), or (j) is used as the gene encoding an enzyme having butyraldehyde dehydrogenase activity.
(f) A DNA comprising the nucleotide sequence of SEQ ID NO: 11
(g) A DNA comprising a nucleotide sequence that has 90% or more identity to the nucleotide sequence of SEQ ID NO: 11 and encoding a polypeptide having butyraldehyde dehydrogenase activity
(h) A DNA encoding a polypeptide comprising the amino acid sequence of SEQ ID NO: 12
(i) A DNA encoding a polypeptide comprising an amino acid sequence that has 90% or more identity to SEQ ID NO: 12, the polypeptide having butyraldehyde dehydrogenase activity
(j) A DNA encoding a polypeptide comprising an amino acid sequence that has 1 to 100 amino acids deleted, substituted, inserted, or added in the amino acid sequence of SEQ ID NO: 12, the polypeptide having butyraldehyde dehydrogenase activity

4. The transformant according to claim 1 or 2, wherein the following DNA (k), (l), (m), (n), or (o) is used as the gene encoding an enzyme having butanol dehydrogenase activity.
(k) A DNA comprising the nucleotide sequence of SEQ ID NO: 13, 15, 17, or 19
(l) A DNA comprising a nucleotide sequence that has 90% or more identity to the nucleotide sequence of SEQ ID NO: 13, 15, 17, or 19 and encoding a polypeptide having butanol dehydrogenase activity
(m) A DNA encoding a polypeptide comprising the amino acid sequence of SEQ ID NO: 14, 16, 18, or 20
(n) A DNA encoding a polypeptide comprising an amino acid sequence that has 90% or more identity to SEQ ID NO: 14, 16, 18, or 20, the polypeptide having butanol dehydrogenase activity
(o) A DNA encoding a polypeptide comprising an amino acid sequence that has 1 to 100 amino acids deleted, substituted, inserted, or added in the amino acid sequence of SEQ ID NO: 14, 16, 18, or 20, the polypeptide having butanol dehydrogenase activity

5. The transformant according to claim 1 or 2, wherein the *Hydrogenophilus* bacterium is *Hydrogenophilus thermoluteolus.*

6. A method for producing butanol, comprising a step of culturing the transformant according to claim 1 or 2 using substantially only carbon dioxide as a carbon source.
